# EUROPEAN PATENT SPECIFICATION

(11) **EP 3 439 712 B1**
(45) Date of publication and mention of the grant of the patent: **01.10.2025**
(21) Application number: 17718246.6
(22) Date of filing: 04.04.2017
(51) Int. Cl.: A61K 31/19, A61K 31/7004, A61K 31/715, A61K 33/14, A61M 1/16, A61M 1/28, C02F 1/28, C02F 1/32, C02F 1/42, C02F 1/44

(54) **PERITONEAL DIALYSATE FLUID GENERATION SYSTEM WITH INTEGRATED CYCLER**
SYSTEM ZUR ERZEUGUNG EINER PERITONEALDIALYSATFLÜSSIGKEIT MIT INTEGRIERTEM CYCLER
SYSTÈME DE GÉNÉRATION DE FLUIDE DE DIALYSAT PÉRITONÉAL DOTÉ D'UN CYCLEUR INTÉGRÉ

(30) Priority: 04.04.2016 US 201662318173 P
(43) Date of publication of application: 13.02.2019
(73) Proprietor: Mozarc Medical US LLC, Minneapolis, MN 55431 (US)
(72) Inventor: MANDA, VenKatesh R., Stillwater Minnesota 55082 (US); GERBER, Martin T., Maple Grove Minnesota 55369 (US); HOBOT, Christopher M., Rogers Minnesota 55374 (US); LURA, David B., Maple Grove Minnesota 55311-2672 (US); MEYER, Thomas E., Stillwater Minnesota 55082 (US)
(74) Representative: Maschio & Soames IP Ltd
(86) International application number: PCT/US2017/025876
(87) International publication number: WO 2017/176701

(56) References cited:
- WO-A1-00/57928
- WO-A1-00/57935
- US-A- 4 747 822
- US-A- 5 032 265
- US-A- 5 643 201
- US-A1- 2013 168 316
- US-A1- 2014 018 727
- JENKINS S. ET AL: "Mixing osmotic agents - two different approaches", PERITONEAL DIALYSIS INTERNATIONAL, vol. 27, no. 3, 1 May 2007 (2007-05-01), pages 245 - 250, XP055865708

## Description

### FIELD OF THE INVENTION

The invention relates to devices, systems, and methods for generating a peritoneal dialysate having purity and sterility characteristics suitable for Peritoneal Dialysis (PD). The peritoneal dialysate can be generated from water of variable quality using a dialysate generation flow path containing a sterilization module. The sterilization module can be any one or more of an ultrafilter, Ultraviolet (UV) light source, microbial filter, dialyzer, and combinations thereof. Peritoneal dialysate generation system and related methods are described that can automatically generate peritoneal dialysate and deliver peritoneal dialysis therapy to a patient using an integrated cycler for delivering the peritoneal dialysate.

### BACKGROUND

Peritoneal Dialysis (PD), including Automated Peritoneal Dialysis (APD) and Continuous Ambulatory Peritoneal Dialysis (CAPD), is a dialysis treatment that can be performed at home, either by the patient alone or with a care-giver. PD differs from Hemodialysis (HD) in that blood is not removed from the body and passed through a dialyzer, but rather a catheter is placed in the peritoneal cavity and dialysate introduced directly into the peritoneal cavity. Blood is cleaned inside the patient using the patient's own peritoneum as a type of dialysis membrane. However, because fluid is directly introduced into a human body, the fluid used for peritoneal dialysate is generally required to be free of biological and chemical contaminants. The peritoneal dialysate should also contain specified concentrations of solutes and cations for biocompatibility and for performing membrane exchange.

Peritonitis is a serious and common problem in the PD population that results in abdominal pain, fever, and cloudy dialysate. Peritonitis remains a leading complication of PD with around 18% of infection-related mortality in PD patients resulting from peritonitis (Fried et al., "Peritonitis influences mortality in peritoneal dialysis patients," J. Am. Soc. Nephrol. 1996; 7:2176-2182). Moreover, peritonitis is a contributing factor to death in 16% of deaths on PD, and remains a major cause for patients discontinuing PD and switching to HD. Peritonitis and other peritoneal dialysis complications can often be traced to non-sterile techniques and/or contaminated starting dialysate.

The US FDA regulates pre-packaged dialysate for use in PD as a Class II drug if the pre-packaged dialysate is used in either a semi-automatic PD system or an automatic PD system *(e.g.,* cycler system). *See* 21 C.F.R. Sec. 876.5630. If the peritoneal dialysate is not pre-packaged, the US FDA requires the dialysate be prepared from a dialysate concentrate and "sterile purified water," which is defined by the FDA in 21 C.F.R. Sec. 165.110(a)(2)(iv) and (vii). Some possible contaminants present in water used to prepare dialysis fluid can be (i) particles, (ii) chemicals, and (iii) microbial contaminants such as bacteria, fungi and yeasts, and microbial derivatives or fragments (e.g., endotoxins released during active growth and lysis of micro-organisms). In additional to meeting purity and sterility requirements, peritoneal dialysate must also contain specific and precise amounts of solutes, such as sodium chloride, sodium bicarbonate, osmotic agents, buffers, and cation infusates.

Because traditional peritoneal dialysis systems require FDA-approved, pre-packaged dialysate, the dialysate can be expensive due to high manufacturing, shipping, and storage costs. Shortages can also occur. The problems are not mitigated by on-site dialysate preparation because the source water must still meet high fluid purity and sterility characteristics. Such standards may be difficult to meet, particularly for continuous, automatic peritoneal dialysis machines designed for home use. Further, traditional systems usually require storage of hundreds of liters of dialysate bags, including 300 L or more of peritoneal dialysate and over 300 kg of fluid per month. Storage and shipping of the peritoneal dialysate is expensive, labor intensive, and requires significant storage space.

Known systems and methods require significant space to store peritoneal dialysate prior to use. Continuous ambulatory peritoneal dialysis (CAPD) traditionally uses 1-4 exchanges of peritoneal dialysate a day, with an overnight dwell. Because each exchange requires approximately 2-4 L of peritoneal dialysate, use of prepackaged dialysate requires storing about 8-16 L of dialysate per day, or 56-112 L of dialysate per week. Automated peritoneal dialysis uses a cycler to cycle peritoneal dialysis into and out of the peritoneal cavity of the patient, generally at night. APD generally uses 3-5 exchanges daily, requiring up to 20 L of dialysate per day and up to 140 L of dialysate per week. Tidal Peritoneal Dialysis (TPD) is similar to APD with the exception that a between 250 mL to 1000 mL of the peritoneal dialysate is left in the peritoneal cavity of the patient between infusions. The known systems and methods require significant storage space and can deter the adoption of CAPD, APD, or TPD.

There is a need for systems and methods that can generate and use peritoneal dialysate using water of varying quality. There is also a need for a system that can generate peritoneal dialysate and use the peritoneal dialysate with an integrated cycler, reducing the number of components necessary for peritoneal dialysis. The need includes peritoneal dialysate having purity and sterility requirements such that patients will not contract an infection due to bacteria or other pathogens in fluid used for peritoneal dialysate. The need is acute for automated fluid generation for continuous dialysis machines for use at home where a water source can be tap water or other non-sterile source. There is also a need for systems and methods that allow for the automated generation of dialysate suitable for peritoneal dialysis that contains the proper amounts of solutes and cations.

There is further a need for a system that uses filtration, as opposed to heat, in sterilization of the dialysate, which reduces the generation of glucose degradation products. There is also a need for a system that can generate peritoneal dialysate on demand, or for direct infusion into the patient, reducing the storage time and space requirements, as well as lowering the probability of loss of sterility of the dialysate. The paper by Jenkins S et al "Mixing Osmotic Agents - two different approaches", Peritoneal Dialysis International" vol 27. no. 3, 1 May 2007 (2007-05-01) pages 245-250 XP-55865708, describes dialysates made up of combinations of osmotic agents, such as glucose and icodextrin. PCT Application WO 00/57928 describes a device to produce medical fluids and solutions such as blood component handling, including glucose which may be replaced or supplemented with glucose polymers.

### SUMMARY OF THE INVENTION

The invention is set out in the appended set of claims.

### BRIEF DESCRIPTION OF THE DRAWINGS

FIG. 1 shows a peritoneal dialysate generation flow path with an integrated cycler.
FIG. 2 shows a system for adding concentrates to a peritoneal dialysate generation flow path.
FIG. 3 shows an overview of a system for generating and using peritoneal dialysate with a single concentrate source.
FIG. 4 shows an overview of a system for generating and using peritoneal dialysate with multiple concentrate sources.
FIG. 5 shows an alternative peritoneal dialysate generation flow path with an integrated cycler.
FIG. 6 shows a peritoneal dialysate generation flow path with multiple dispensing options.
FIG. 7A shows a perspective view of a peritoneal dialysate generation cabinet with an integrated cycler.
FIG. 7B shows a front view of a peritoneal dialysate generation cabinet with an integrated cycler.
FIG. 7C shows a peritoneal dialysate generation cabinet with the doors closed.
FIG.'s 8A-D show a peritoneal dialysate generation cabinet with a water reservoir and waste reservoir.
FIG. 9 shows a peritoneal dialysate generation cabinet connected to a faucet and drain.
FIG. 10 shows a dialysis caddy for use in a peritoneal dialysate generation flow path.

### DETAILED DESCRIPTION OF THE INVENTION

Unless defined otherwise, all technical and scientific terms used have the same meaning as commonly understood by one of ordinary skill in the art.

The articles "a" and "an" are used to refer to one or to over one *(i.e.,* to at least one) of the grammatical object of the article. For example, "an element" means one element or over one element.

"Activated carbon" refers to a form of carbon processed to have small pores, increasing the surface area available for adsorption.

The term "amino acid," as used herein, refers to any nitrogen containing organic acid or peptide that can be used as an osmotic agent to generate peritoneal dialysate.

The term "calcium chloride source" refers to a source of calcium chloride in solid and/or solution form. The calcium chloride source can contain at least one fluid pathway and include components such as conduits, valves, filters or fluid connection ports, any of which are fluidly connectable to each other or to a fluid flow path. The calcium chloride source can either be formed as a stand-alone enclosure or a compartment integrally formed with an apparatus for dialysis for containing the calcium chloride source.

A "carbon filter" is a bed of activated carbon.

The term "comprising" includes, but is not limited to, whatever follows the word "comprising." Use of the term indicates the listed elements are required or mandatory but that other elements are optional and may be present.

A "concentrate pump" is a pump configured to move fluid between a concentrate source and a flow path.

A "concentrate solution" is a solution of one or more solutes in water. The concentrate solution can have a solute concentration greater than that to be used in dialysis.

A "concentrate source" is a source of one or more solutes. The concentrate source can have one or more solutes that has a solute concentration greater than the solute concentration to be used for dialysis.

A "conductivity sensor" is device for measuring the electrical conductance of a solution and/or the ion, such as a sodium ion, concentration of a solution.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The terms "control," "controlling," or "controls" refers to the ability of one component to direct the actions of a second component.

A "control system" can be a combination of components acting together to maintain a system to a desired set of performance specifications. The control system can use processors, memory and computer components configured to interoperate to maintain the desired performance specifications. The control system can also include fluid or gas control components, and solute control components as known within the art to maintain the performance specifications.

The terms "controlled addition," to "control addition," or "controlling addition" refer to the ability to add one or more substances or fluids to a flow path or container in an accurately controllable amount.

The phrase "controlling the movement of fluid" refers to directing fluid through a flow path, container, receptacle, or reservoir of any type.

The term "dextrose source" refers to a source of dextrose in solid and/or solution form. The dextrose source can interface with at least one other module found in systems for dialysis. The dextrose source can contain at least one fluid pathway and include components such as conduits, valves, filters or fluid connection ports, any of which are fluidly connectable to each other or to a fluid flow path. The dextrose source can either be formed as a stand-alone enclosure or a compartment integrally formed with an apparatus for dialysis for containing a dextrose source.

The term "dialysate" describes a fluid into or out of which solutes from a fluid to be dialyzed diffuse through a membrane. Dialysate can differ depending on the type of dialysis to be carried out. For example, dialysate for peritoneal dialysis may include different solutes or different concentrations of solutes than dialysate for hemodialysis.

A "dialysate container" is any container capable of storing or containing dialysate for dialysis. The container any be of any suitable, size, geometry, or configuration.

The term "dialysis caddy" refers to a container detachably removable from a dialysis system, the caddy configured to hold one or more other containers. In any embodiment, the caddy can include one or more connectors for fluid connection from the containers to the dialysis system.

The term "downstream" refers to a position of a first component in a flow path relative to a second component wherein fluid will pass by the second component prior to the first component during normal operation. The first component can be said to be "downstream" of the second component, while the second component is "upstream" of the first component.

A "drain line" is a fluid line for carrying fluid to a drain such as a waste receptacle or drain. The drain line can be connected to a peritoneal cavity of a patient for draining fluid.

The term "filter" refers to a porous component through which fluid can pass, but that traps one or more materials within the fluid.

A "fitting feature" is any protrusion, indentation, groove, ridge, having any shape, size, or geometry that ensures that only a corresponding fitting feature complementary to the fitting feature can form a connection or fit to the corresponding fitting feature. The fitting feature can also include non-mechanical means for ensuring complementary connection such as magnets placed at particular locations, or visual or aural indicators such as color, lettering, or sound. The fitting feature can be affixed, integral, or labeled on a component or surface to ensure a corresponding feature on a desired component or surface can mate or connect to the component or surface having the fitting feature.

A "flash pasteurization module" is a component or set of components capable of heating a fluid to a high temperature and recirculating the fluid for sterilization.

A "flow meter" is a device capable of measuring an amount or rate of fluid moving past or through a particular location.

The term "fluid" can be any substance without a fixed shape that yields easily to external pressure such as a gas or a liquid. Specifically, the fluid can be water containing any solutes at any concentration. The fluid can also be dialysate of any type including fresh, partially used, or spent.

The terms "fluid connection," "fluidly connectable," or "fluidly connected" refer to the ability to pass fluid or gas from one point to another point. The two points can be within or between any one or more of compartments, modules, systems, and components, all of any type.

The terms "to generate peritoneal dialysate" or "peritoneal dialysate generation" refers to creating a peritoneal dialysate solution from constituent parts.

The term "glucose source" refers to a source of glucose in solid and/or solution form. The glucose source can interface with at least one other module found in systems for dialysis. The glucose source can contain at least one fluid pathway and include components such as conduits, valves, filters or fluid connection ports, any of which are fluidly connectable to each other or to a fluid flow path. The glucose source can either be formed as a stand-alone enclosure or a compartment integrally formed with an apparatus for dialysis for containing a glucose source.

A "heater" is a component capable of raising the temperature of a substance, container, or fluid.

The terms "heating" or to "heat" refer to raising the temperature of a substance, fluid, or container.

An "integrated cycler" is a component for movement of fluid into and out of the peritoneal cavity of a patient, wherein the integrated cycler forms a part of an overall system. For example, the integrated cycler can be contained in a housing with other components used for peritoneal dialysis and be in fluid and electrical connection with desired components.

An "infusion line" is a fluid line for carrying peritoneal dialysate into a body cavity or part of a patient such as a peritoneal cavity.

An "ion concentrate" refers to one or more ionic compounds. The ion concentrate can have one or more ionic compounds in the ion concentrate. Further, the ion concentrate can have an ion concentration greater than an ion concentration to be used in dialysis.

An "ion concentrate source" refers to a source of one or more ionic compounds. The ion concentrate source can be in water or solid form. The ion concentrate source can further have one or more ionic compounds that are at a higher ion concentration greater than generally used in dialysis.

The term "level of sterility" refers to an estimated probability of viable organisms surviving a sterilization process.

The term "magnesium chloride source" refers to a source of magnesium chloride in solid and/or solution form. The magnesium chloride source can interface with at least one other module found in systems for dialysis. The magnesium chloride source can contain at least one fluid pathway and include components such as conduits, valves, filters or fluid connection ports, any of which are fluidly connectable to each other or to a fluid flow path. The magnesium chloride source can either be formed as a stand-alone enclosure or a compartment integrally formed with an apparatus for dialysis for containing a magnesium chloride source.

The term "microbial filter" refers to a device inhibiting passage of microbes or fragments of microbes such as endotoxins in a fluid or solution while allowing the passage of the fluid or solution.

A "nanofilter" is a filter membrane having nanometer sized pores.

An "osmotic agent" is a substance dissolved in water capable of driving a net movement of water by osmosis across a semi-permeable membrane due to concentration differences of the osmotic agent on each side of the semi-permeable membrane.

An "osmotic agent source" refers to a source of osmotic agents in solid and/or solution form. The osmotic agent source can interface with at least one other module found in systems for dialysis. The osmotic agent source can contain at least one fluid pathway and include components such as conduits, valves, filters or fluid connection ports, any of which are fluidly connectable to each other or to a fluid flow path. The osmotic agent source can either be formed as a stand-alone enclosure or a compartment integrally formed with an apparatus for dialysis for containing an osmotic agent source.

The term "peritoneal cavity" refers to the space between the parietal peritoneum and visceral peritoneum of a patient.

"Peritoneal dialysate" is a dialysis solution to be used in peritoneal dialysis having specified parameters for purity and sterility. Peritoneal dialysate is not the same as dialysate used in hemodialysis although peritoneal dialysate may be used in hemodialysis.

A "peritoneal dialysate generation flow path" is a path used in generating dialysate suitable for peritoneal dialysis.

A "peritoneal dialysate generation system" refers to a collection of components used to generate peritoneal dialysate.

"Peritoneal dialysis" is a therapy wherein a dialysate is infused into the peritoneal cavity, which serves as a natural dialyzer. In general, waste components diffuse from a patient's bloodstream across a peritoneal membrane into the dialysis solution via a concentration gradient. In general, excess fluid in the form of plasma water flows from a patient's bloodstream across a peritoneal membrane into the dialysis solution via an osmotic gradient. Once the infused peritoneal dialysis solution has captured sufficient amounts of the waste components the fluid is removed. This cycle can be repeated for several cycles each day or as needed.

A "predetermined time" is a set time for an event to occur, such as a set time of day, or a set length of time from a previous event.

The term "prescribed solute concentration" refers to the concentration of one or more solutes in peritoneal dialysate intended for use by a patient.

The term "pressure sensor" refers to a device for measuring the pressure of a gas or liquid in a vessel, container, or fluid line.

The term "pump" refers to any device that causes the movement of fluids or gases by applying suction or pressure.

The terms "pumping fluid" or to "pump fluid" refer to moving a fluid through a flow path with a pump.

A "purified water source" is a water source containing purified water.

"Purified water" can be defined as water produced by distillation, deionization, reverse osmosis, or other suitable processes and meets the definition of "purified water" in the United States Pharmacopeia, 23d Revision, January 1, 1995, and the FDA at 21 CFR Section §165.110(a)(2)(iv). Other criteria for purified water can be determined by those of skill in the art, particularly as relating to purified water suitable for peritoneal dialysis.

A "reverse osmosis module" is a set of components to drive fluid through one or more semipermeable membranes, wherein pressure is used to move the fluid from a side of the semipermeable membrane with a higher concentration of one or more solutes to a side of the semipermeable membrane with a lower concentration of the one or more solutes.

A "selected time" is a set time chosen by a user or algorithm.

The term "sodium chloride source" refers to a source of sodium chloride in solid and/or solution form. The sodium chloride source can interface with at least one other module found in systems for dialysis. The sodium chloride source can contain at least one fluid pathway and include components such as conduits, valves, filters or fluid connection ports, any of which are fluidly connectable to each other or to a fluid flow path. The sodium chloride source can either be formed as a stand-alone enclosure or a compartment integrally formed with an apparatus for dialysis for containing a sodium chloride source.

The term "sodium lactate source" refers to a source of sodium lactate in solid and/or solution form. The sodium lactate source can interface with at least one other module found in systems for dialysis. The sodium lactate source can contain at least one fluid pathway and include components such as conduits, valves, filters or fluid connection ports, any of which are fluidly connectable to each other or to a fluid flow path. The sodium lactate source can either be formed as a stand-alone enclosure or a compartment integrally formed with an apparatus for dialysis for containing a sodium lactate source.

A "solute" is a substance dissolved in a solvent, such as water.

The term "sorbent cartridge" refers to a cartridge containing one or more sorbent materials for removing specific solutes from solution. The term "sorbent cartridge" does not require the contents in the cartridge be sorbent based, and the contents of the sorbent cartridge can be any contents capable of removing solutes from a dialysate. The sorbent cartridge may include any suitable amount of one or more sorbent materials. In certain instances, the term "sorbent cartridge" refers to a cartridge which includes one or more sorbent materials besides one or more other materials capable of removing solutes from dialysate. "Sorbent cartridge" can include configurations where at least some materials in the cartridge do not act by mechanisms of adsorption or absorption.

A "sterilization module" is a component or set of components to sterilize a fluid by removing or destroying chemical or biological contaminants.

A "temperature sensor" is a sensor capable of determining the temperature of a fluid.

A "timer" is a device capable of determining the time of day, or the time elapsed between multiple events.

An "ultrafilter" is a semi permeable membrane through which a fluid can pass with removal of one or more solutes or particles from the fluid.

The term "upstream" refers to a position of a first component in a flow path relative to a second component wherein fluid will pass by the first component prior to the second component during normal operation. The first component can be said to be "upstream" of the second component, while the second component is "downstream" of the first component.

A "user interface" is a component that allows a user to communicate information or instructions to a processor or a memory device and to receive information or instructions from the processor or memory device.

A "UV light source" is a component which uses ultraviolet light to kill biological contaminants in a fluid.

A "valve" is a device capable of directing the flow of fluid or gas by opening, closing or obstructing one or more pathways to allow the fluid or gas to travel in a path. One or more valves configured to accomplish a desired flow can be configured into a "valve assembly."

A "waste reservoir" is a container for collecting and storing used or waste fluids.

The term "water purification module" refers to a component or components capable of removing biological or chemical contaminants from water.

The term "water source" refers to a source from which potable water can be obtained.

### Peritoneal Dialysis System with an Integrated Cycler

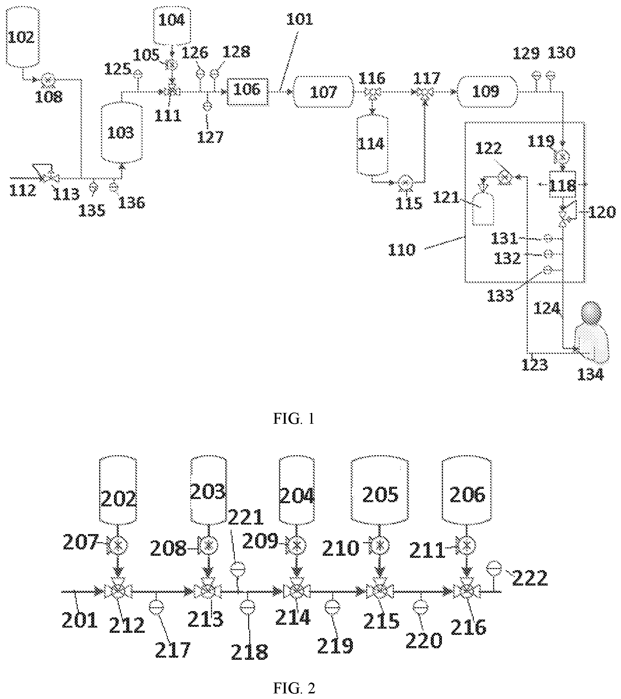

A system for generating peritoneal dialysate and delivering peritoneal dialysis therapy to a patient **134** can be configured as illustrated in FIG. 1. This example does not form part of the claimed invention. The system includes a peritoneal dialysate generation flow path **101.** Fluid from a water source, such as water tank **102,** can be pumped into the peritoneal dialysate generation flow path **101.** Additionally, or as an alternative to a water tank **102,** the system can use a direct connection **112** to a water source. System pump **108** can control the movement of fluid through the peritoneal dialysate generation flow path **101.** If a direct connection **112** to a water source is used, a pressure regulator **113** ensures the incoming water pressure is within a predetermined range. The system pumps the fluid from water source through a water purification module **103** to remove chemical contaminants in the fluid in preparation for creating dialysate.

In any embodiment of the first or second aspects of the invention, the water source can be a source of potable water including a purified water source. Purified water can refer to water that meets the definition of "purified water" in the United States Pharmacopeia, 23d Revision, January 1, 1995. Alternatively, purified water can refer to any source of water treated to remove at least some biological or chemical contaminants, whether or not the water meets the definition of purified water in United States Pharmacopeia, 23d Revision, January 1, 1995. In any embodiment of the first or second aspects of the invention, the water tank **102** can be a non-purified water source, such as tap water, wherein the water from the water tank **102** can be purified by the system as described. A non-purified water source can provide water that has undergone no additional purification, such as tap water from a municipal water source, water that has undergone some level of purification, but does not meet the definition of "purified water" provided, such as bottled water or filtered water. In any embodiment, the water source can contain water meeting the WHO drinkable water standards provided in Guidelines for Drinking Water Quality, World Health Organization, Geneva, Switzerland, 4th edition, 2011. The peritoneal dialysate generation flow path **101** can also have a direct connection **112** to a purified or non-purified water source, shown as direct connection **112.** The water source can be any source of water, whether from a tap, faucet, or a separate container or reservoir.

In any embodiment of the first or second aspects of the invention, the water purification module 103 can be a sorbent cartridge. The sorbent cartridge includes an anion exchange material such as zirconium oxide. The zirconium oxide can remove anionic species from the fluid, such as phosphate or fluoride molecules, replacing the anionic species with acetate or hydroxide ions. The sorbent cartridge can have any anion exchange material known in the art capable of removing anionic species from the fluid. In any embodiment, the sorbent cartridge can include aluminum oxide for removal of fluoride and heavy metals. In any embodiment, the sorbent cartridge can have a first layer of aluminum oxide, a second layer of activated carbon and a third layer of an ion exchange resin. Alternatively, the water purification module 103 can be a combination of ion and anion exchange materials. The sorbent cartridge can be sized depending on the needs of the user, with a larger sized sorbent cartridge allowing for more exchanges before the sorbent cartridge must be replaced. The sorbent cartridge can include a cation exchange material, such as zirconium phosphate. The zirconium phosphate can remove cationic species from the fluid, such as potassium, calcium, magnesium, or other cations, replacing the cationic species with hydrogen or sodium. The sorbent cartridge can include any cation exchange material capable of removing cations from the fluid. The sorbent cartridge can also include activated carbon. The activated carbon operates to adsorb non-ionic molecules, organic molecules, and chlorine from the water, along with some endotoxins or bacterial contaminants. The activated carbon can be present in the sorbent cartridge in the form of a carbon filter or pad, or as a material layer in the sorbent cartridge. A carbon filter or pad is a bed of activated carbon. The carbon filter can be in a self-contained packaging, or present as a layer of activated carbon within the sorbent cartridge. The sorbent cartridge can purify up to 3 L of water per exchange for a single infusion, with flow rates of up to 300 ml/min. A larger sorbent cartridge can be used when generating peritoneal dialysate for multiple infusions, including a sorbent cartridge that can purify between 3 and 20 L, between 3 and 5 L, between 3 and 10 L, between 5 and 12 L, between 10 and 15 L, or between 10 and 20 L of water, or more.

In any embodiment, the sorbent cartridge can be a single use component or a rechargeable component. Recharging can refer to the process of treating a sorbent material to restore the functional capacity of the sorbent material so as to put the sorbent material back into a condition for use or reuse in a new dialysis session. In some instances, recharging also includes treating a sorbent material so as to clean the sorbent material so that the sorbent material can be stored and used in a subsequent dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged."

The sorbent cartridge can additionally include a microbial filter and/or a particulate filter. A microbial filter can further reduce the amount of endotoxins or bacterial contaminants present in the fluid from the water tank **102** or direct connection **112**. A particulate filter can remove particulate matter from the fluid. The water tank **102** can be any size usable with the system, including between 12 and 20 L. A water tank **102** of 15 L can generally generate the necessary peritoneal dialysate for multiple cycles.

Alternatively, the water purification module **103** can be any component capable of removing contaminants from the water in the water source, including any one or more of a sorbent cartridge, reverse osmosis module, nanofilter, combination of ion and anion exchange materials, activated carbon, silica, or silica based columns.

After the fluid passes through the water purification module **103,** the fluid is pumped to a concentrate source **104,** where necessary components for carrying out peritoneal dialysis can be added from the concentrate source **104.** The concentrates in the concentrate source **104** are utilized to create a peritoneal dialysis fluid that matches a dialysis prescription. Concentrate pump **105** and concentrate valve **111** can control the movement of concentrates from the concentrate source **104** to the peritoneal dialysate generation flow path **101** in a controlled addition. Concentrate valve **111** can be replaced with a hose T. A hose T is a fluid connector in a T-shape, with a port at each end for fluid to enter or exit the hose T. The concentrates added from the concentrate source **104** to the peritoneal dialysate generation flow path **101** can include any component prescribed for use in peritoneal dialysate. Table 1 provides non-limiting exemplary ranges of commonly used components of peritoneal dialysate.

**Table 1**

| **Component** | **Concentration** |
|---|---|
| Sodium chloride | 132-134 mmol/L |
| Calcium chloride dehydrate | 1.25-1.75 mmol/L |
| Magnesium chloride hexahydrate | 0.25-0.75 mmol/L |
| Sodium Lactate | 35-40 mmol/L |
| Dextrose (D-glucose) monohydrate | 0.55-4.25 g/dL |
| pH | 5-6 |
| Osmolality | 346-485 (hypertonic) |

To reduce the glucose degradation products (GDP) formed, some peritoneal dialysate systems use a low GDP formulation. Exemplary peritoneal dialysate concentrations for low GDP formulations are provided in Table 2. Generally, the low GDP peritoneal dialysate is provided in two separate bags, with one bag containing calcium chloride, magnesium chloride and glucose maintained at low pH, and the second bag containing sodium chloride and the buffer components, including sodium lactate and sodium bicarbonate. The two bags are mixed prior to use to generate a peritoneal dialysate with a neutral pH. Alternatively, a two chamber bag is used, the chambers separated by a divider which is broken to mix the fluids prior to use.

**Table 2**

| Low GDP peritoneal dialysate formulations | |
|---|---|
| **Component** | **Concentration** |
| Sodium | 132-134 mEq/L |
| Calcium | 2.5-3.5 mEq/L |
| Magnesium | 0.5-1.0 mEq/L |
| Lactate | 0-40 mEq/L |
| Bicarbonate | 0-34 mEq/L |
| pH | 6.3-7.4 |
| % glucose (g/dL) | 1.5-4.25 |

One of skill in the art will understand that other components can be used in place of the components listed in Tables 1-2. For example, dextrose as listed in Table 1 is commonly used as an osmotic agent. In any embodiment of the first or second aspects of the invention, other osmotic agents can be used in addition to, or in place of, the dextrose, including glucose, icodextrin or amino acids, including dialysate with multiple osmotic agents. Although the sources of sodium, calcium, and magnesium listed in Table 1 are chloride salts, other sodium, magnesium, and calcium salts can be used, such as lactate or acetate salts. Peritoneal dialysate may also contain buffers for maintaining pH of the peritoneal dialysate. Exemplary, non-limiting examples of suitable buffers include bicarbonate buffer, acetate buffer, or lactate buffer. Although not generally used in peritoneal dialysis, potassium chloride can be used for hypokalemic patients who don't receive sufficient potassium through diet. The concentrate source **104** can contain one or more osmotic agents, as well as one or more ion concentrates, such as concentrated sodium chloride, sodium lactate, magnesium chloride, calcium chloride, and/or sodium bicarbonate. The concentrate source **104** can be a single source of concentrates, including both osmotic agents and ion concentrates, or can include multiple sources of concentrates, with separate sources for the osmotic agents and ion concentrates. In any embodiment, the system can have a single concentrate that has all components mixed for a daytime or overnight treatment for use in a home by a single patient. Alternatively, the concentrate source **104** can include separate sources for any one or more of the solutes to be used in the peritoneal dialysate each with a separate concentrate pump to add each component needed to create the peritoneal dialysate. Concentrate pump **105** pumps concentrated solutions from the concentrate source or sources **104** to the peritoneal dialysate generation flow path **101** in a controlled addition. Where more than one concentrate source is used, separate concentrate pumps can move each of the concentrates into the peritoneal dialysate generation flow path **101,** or a single concentrate pump can be used, with valves configured allow individual control over the movement of each of the concentrate solutions to the peritoneal dialysate generation flow path **101.**

After addition of solutes from the concentrate source **104,** the fluid in the peritoneal dialysate generation flow path **101** can contain all the necessary solutes for peritoneal dialysis. The peritoneal dialysate should reach a level of sterility for peritoneal dialysis. The level of sterility can be any level that meets an applicable regulatory requirement, such as a sterility assurance level of 10⁻⁶ required by the FDA, meaning that the chance a viable organism is present after sterilization is 1 in 1,000,000. The system can pump the fluid to a sterilization module for sterilization of the peritoneal dialysate. As shown in FIG. 1, the sterilization module can include one or more of a first ultrafilter **107,** a second ultrafilter **109,** and a UV light source **106.** The sterilization module can be any component or set of components capable of sterilizing the peritoneal dialysate. In any embodiment, the sterilization module can be a single or multiple ultrafilters. A secondary component, such as a UV light source **106** or microbial filter (not shown), can be used in the sterilization module to provide additional sterilization of the peritoneal dialysate. The sterilization module can also include a microbial filter (not shown in FIG. 1). The sterilization module can also include at least two ultrafilters, including second ultrafilter **109** for further sterilization of the fluid and redundancy of the system to protect against sterilization failure. The UV light source **106** can be positioned at any location in the peritoneal dialysate generation flow path **101,** including upstream of ultrafilter **107,** between ultrafilters **107** and **109** or downstream of ultrafilter **109.** The ultrafilters **107** and **109** used in the sterilization module can be replaced as necessary. In any embodiment, the ultrafilters **107** and **109** can have a 3-6 month lifetime before replacement. The ultrafilters **107** and **109** can be any ultrafilter known in the art capable of sterilizing the peritoneal dialysate. A non-limiting example of an ultrafilter that can be used in the systems described is the hollow fiber ForClean ultrafilter, available from Bellco, Mirandola (MO), Italy. In certain embodiments, the sterilization module **106** can use heat sterilization. The sterilization module can include a heater (not shown) to heat the prepared dialysate. Alternatively or additionally, the sterilization module can include a flash pasteurization module (not shown) to sterilize the dialysate through flash pasteurization. The sterilization module can include both heat-based sterilization components and filtration based sterilization components, with the user adjusting the mode of sterilization based on the mode of use. For example, a heat based sterilization can be used when the peritoneal dialysate is generated for later use, while a filtration based sterilization can be used when the peritoneal dialysate is generated for immediate use.

The generated peritoneal dialysate can be pumped directly to an integrated cycler **110** for immediate infusion into a patient **134.** Alternatively, the dialysate can be pumped to an optional dialysate container **114** as a pre-prepared bolus of solution for storage until ready for use by a patient **134.** Valve **116** can control the movement of fluid to either the integrated cycler **110** or the dialysate container **114.** Stored dialysate in dialysate container **114** can be pumped as needed to the integrated cycler **110** by pump **115** through valve **117.** The dialysate container **114** can include one or more sterilized dialysate bags. The dialysate bags, once filled with peritoneal dialysate, can be stored until needed by the patient **134.** The dialysate container **114** can alternatively be a reusable sterilized container or bag. The reusable container or bag can be cleaned and sterilized daily, or at set time periods. Alternatively, the dialysate container **114** can be any type of storage container, such as a stainless steel container. The dialysate container **114** can store enough peritoneal dialysate for a single infusion of peritoneal dialysate into the patient **134,** or enough peritoneal dialysate for multiple infusions into a patient **134.** Additional or alternative storage containers can be included at other locations in the peritoneal dialysate generation flow path **101.** A storage container can be included upstream of the sterilization module, and downstream of the water purification module **103.** Before the fluid is utilized in the cycler stage, the fluid can be pumped through the sterilization module to ensure sterility of stored fluid. Further, concentrates can be added to fluid before storing the fluid, or after storage of the fluid but prior to sterilization in the sterilization module.

The storage containers can be either upstream or downstream of the concentrate source **104.** The addition of concentrates to the fluid can happen either before storage of the fluid, or after storage of the fluid just before sterilization in the sterilization module.

By generating and immediately using the peritoneal dialysate, the dialysate storage time can be reduced, reducing the possibility of bacterial growth. A user interface can be included on the peritoneal dialysis generation machine in communication with the control system, allowing a patient **134** to direct the generation of peritoneal dialysate at a selected time as needed. Additionally, or alternatively, the peritoneal dialysate machine can include a timer, and the timer can cause the peritoneal dialysate machine to generate peritoneal dialysate at predetermined times according to the patient's **134** peritoneal dialysis schedule. Alternatively, the peritoneal dialysate generation machine can be equipped with wireless communication, such as Wi-Fi, Bluetooth, Ethernet, or any other wireless communication system known in the art. The user can direct the peritoneal dialysis machine to generate peritoneal dialysate at a specified time from any location. By using a timer, user interface, or wireless communication to control the generation of peritoneal dialysate on demand, the peritoneal dialysate storage time can be reduced, lowering the chances of generating significant amounts of degradation products or allowing bacterial growth.

The peritoneal dialysate can be generated and used in real time, with direct infusion of the peritoneal dialysate into the patient **134** through the integrated cycler **110.** For real time generation and use of the peritoneal dialysate, the flow rate of fluid through the peritoneal dialysate generation flow path **101** can be between 50 and 300 ml/min. With the online generation of fluid described, a flow rate of 300 ml/min can support an exchange time of between 10 and 15 minutes for a full cycle of draining and filling the peritoneal cavity of a patient **134.** If a dialysate container **114** is used to store generated peritoneal dialysate, the flow rate of fluid through the peritoneal dialysate generation flow path **101** can be any rate. The integrated cycler **110** can then infuse the generated peritoneal dialysate into the peritoneal cavity of a patient **134.** The integrated cycler **110** and the rest of the system can communicate for the purposes of generation and use of the peritoneal dialysate by any method known in the art, including Bluetooth, Wi-Fi, Ethernet, or direct hardware connections to meet patient or clinic needs. Additional valves and regulators (not shown in FIG. 1) can be included to aid in connection and operation of the peritoneal dialysate generation flow path **101** and integrated cycler **110.** The integrated cycler **110** and the peritoneal dialysate generation flow path **101** can communicate directly, or can each communicate with a control system for control over the generation and use of the peritoneal dialysate.

In any embodiment of the first or second aspects of the invention, the dialysate container **114** can store enough peritoneal dialysate for multiple infusions into the patient **134,** including enough peritoneal dialysate for one day or more of treatment. A timer can be included in the control system and can cause the machine to generate fresh peritoneal dialysate each day or at set times.

The integrated cycler **110** can include a metering pump **119** for metering peritoneal dialysate into the peritoneal cavity of the patient **134.** An in-line heater **118** heats the peritoneal dialysate to a desired temperature prior to infusion into the patient **134.** A pressure regulator **120** ensures the peritoneal dialysate pressure is within a predetermined range safe for infusion into the patient **134.** The metering pump **119** can use any safe pressure for infusing fluid into the patient **134.** Generally, the pump pressures are on average set at ±10.3 kPa or 77.6 mmHg. If there is no fluid flow, the maximum pressure can increase to ±15.2 kPa or 113.8 mmHg for a short period, such as less than 10 seconds. The peritoneal dialysate is infused into the peritoneal cavity of the patient **134** through infusion line **124.** In any embodiment, an additional microbial filter (not shown) may be used to sterilize the peritoneal dialysis fluid immediately before the peritoneal dialysate enters the patient **134.** After a dwell period, the peritoneal dialysate is drained from the patient **134** through drain line **123.** Pump **122** provides a driving force for removing the peritoneal dialysate from the patient **134.** Treatment, other than the first full cycle for a patient in APD, generally begins with drainage of the peritoneal cavity of the patient **134,** prior to infusing the fresh peritoneal dialysate into the patient **134.** An optional waste reservoir **121** can be included to store the used peritoneal dialysate for disposal. Alternatively, the drain line **123** can be directly connected to a drain for direct disposal. A standard waste reservoir **121** is 15 L, however, the waste reservoir **121** can be any size, including between 12 and 20 L. For patients requiring a higher drainage, a drain manifold can be included for connecting multiple waste reservoirs. There is no set rate for draining of peritoneal dialysate from the peritoneal cavity of the patient **134,** and any flow rate can be used with the integrated cycler **110.** A slow flow is defined as a drain flow rate of less than 50 mL/min for a standard fill, and less than 15 mL/min for a low fill. No flow is defined as a drain flow rate of less than 12 mL/min for a standard fill, and less than 3 mL/min for a low fill. If the detected flow rate of the drained dialysate is below the cutoffs, the system can generate an alarm. The fill/drain cycle is typically done in 10 to 15 minutes with 2 to 3 L of fluid moving in total, half of which is moved into the peritoneal cavity and half of which is moved out of the peritoneal cavity. The peritoneal cavity can be drained with a slight negative pressure of about 50 to 100 mbar created by the drain pump **122.** The drain rate can be up to 300 ml/minute or greater and can vary throughout the session. For example, a drain rate can be high such as at 300 ml/min, and then slow, such as to 100ml/min, as the cavity approaches an empty point. Similarly, a fill rate can be as high as 300 ml/min, and also vary throughout a session. In the case of power failure during treatment, the valves and pumps can be closed to prevent any dialysate flow. If power is returned quickly, the therapy can resume. With a longer power failure, an alert can be generated instructing the patient **134** to manually drain the peritoneal dialysate. In any embodiment, a battery backup can be included in the case of power failure. The patient tubing connected to infusion line **124** and drain line **123** can be a consumable or disposable patient tubing set, which can be replaced after each use.

Various sensors positioned in the peritoneal dialysate generation and infusion system ensure that the generated fluid is within predetermined parameters. Flow meter **135** ensures the incoming water is at a correct flow rate, while pressure sensor **136** ensures the incoming water is at an appropriate pressure. Conductivity sensor **125** is used to ensure that the water exiting water purification module **103** has been purified to a level safe for use in peritoneal dialysis. Conductivity sensor **126** ensures the conductivity of the dialysate after the addition of concentrates from concentrate source **104** is within a predetermined range. Refractive index sensor **127** ensures that the concentration of the osmotic agents is within a predetermined range. pH sensor **128** ensures the pH of the peritoneal dialysate is within a predetermined range. After passing through the sterilization module including second ultrafilter **109,** pH sensor **129** and conductivity sensor **130** are used to ensure that no changes in the pH or conductivity have occurred during purification or storage of the dialysate in dialysate container **114.** The integrated cycler **110** has flow meter **131,** pressure sensor **132** and temperature sensor **133** to ensure that the dialysate being infused into the patient **134** is within a proper flow rate, pressure, and temperature range. The flow meter **131** can also calculate the volume of solution infused into the patient **134.** The pressure sensor **132** can monitor the pressure in the peritoneal cavity.

Overfill, or excessive solution in the peritoneal cavity beyond the target volume may present complications in therapy. Overfill can be caused by many factors, including failing to fully drain the peritoneal cavity prior to infusion of fresh peritoneal dialysate. In any embodiment, the integrated cycler **110** can start therapy with a drain step to ensure that no peritoneal dialysate remains in the peritoneal cavity. Monitoring both pressure and volume of peritoneal dialysate introduced to the patient **134** can avoid overfill. If the pressure rises to a certain point, the system can be programmed to end filling or send an alert to a user to complete filling of the peritoneal cavity at a desired level. The volume of peritoneal dialysate extracted from and introduced to the patient **134** can also be monitored with flow meters to ensure proper volumes of exchanges. Draining the peritoneal cavity can be performed in a similar manner by monitoring the pressure and volume of the drained peritoneal dialysate.

As illustrated in FIG. 1, the necessary solutes can be added to the peritoneal dialysate generation flow path **101** from a single concentrate source **104.** The solutes can be present in concentrated from within the concentrate source **104** in a fixed ratio for peritoneal dialysis, as shown in Table 1. Using a single concentrate source **104** for all solutes results in peritoneal dialysate having a fixed ratio of each of the solutes.

Table 3 provides exemplary non-limiting ranges of solutes that can be added from a single concentrate source **104** to the peritoneal dialysate generation flow path **101,** including the starting concentration of the solutes in the concentrate source, as well as exemplary final volumes of the solutes in the dialysate and the exemplary flow rates of both the solutes and the water in the peritoneal dialysate generation flow path 101 that will achieve those concentrations. The solutes shown in Table 3 are traditional peritoneal dialysate solutes. Table 4 shows exemplary ranges of solutes that can be used as a low GDP formulation. Table 5 shows exemplary ranges of solutes that can be used with icodextrin as the osmotic agent. Icodextrin is sometimes used as an osmotic agent for a long dwell period. If dextrose or glucose is used in a long dwell period, reabsorption of the ultrafiltrate can occur, reducing the net volume of fluid removed. Icodextrin results in a long sustained ultrafiltration, and can provide improved ultrafiltration efficiency over a long dwell period. One of skill in the art will understand that the concentrations of any of the solutes shown in Tables 3-5 can be altered by altering the flow rates of the system pump **108** or concentrate pump **105.** However, the ratio of the solutes included is fixed if using a single concentrate source **104.** If the ratio of the solutes needs to be altered for any reason, a new concentrate solution may be needed.

**Table 3**

| Exemplary solutes for addition from a single concentrate source | | | |
|---|---|---|---|
| **Component** | **Concentration (g/l)** | **Solution volume (ml/L)** | **Flow rate (ml/min)** |
| Glucose | 100 - 850 | 50-400 | 1 - 18 |
| Sodium Chloride | 13 - 108 | 50-400 | 1 - 18 |
| Sodium Lactate | 11 - 90 | 50-400 | 1 - 18 |
| MgCl₂.6H₂O | 0.13 - 1.02 | 50-400 | 1 - 18 |
| CaCl₂.2H₂O | 0.6 - 5.1 | 50-400 | 1 - 18 |
| Water | | 600-950 | 50-1000 |

**Table 4**

| Exemplary solute ranges in a low GDP solution | | | |
|---|---|---|---|
| **Component** | **Concentration (g/l)** | **Solution volume (ml/L)** | **Flow rate (ml/min)** |
| Glucose | 100 - 900 | 50-400 | 1 - 18 |
| Sodium Chloride | 13 - 108 | 50-400 | 1 - 18 |
| Sodium Lactate | 11 - 90 | 50-400 | 1 - 18 |
| MgCl₂.6H₂O | 0.13 - 1.02 | 50-400 | 1 - 18 |
| CaCl₂.2H₂O | 0.6 - 5.1 | 50-400 | 1 - 18 |
| Water | | 600-950 | 50-1000 |

**Table 5**

| Exemplary solute ranges in icodextrin solution | | | |
|---|---|---|---|
| **Component** | **Concentration (g/l)** | **Solution volume (ml/L)** | **Flow rate (ml/min)** |
| Icodextrin | 100 - 850 | 100-400 | 2-37 |
| Sodium Chloride | 13 - 108 | 100-400 | 1 - 18 |
| Sodium Lactate | 11 - 90 | 100-400 | 2-37 |
| MgCl₂.6H₂O | 0.13 - 1.02 | 100-400 | 2-37 |
| CaCl₂.2H₂O | 0.6 - 5.1 | 100-400 | 2-37 |
| Water | | 600-900 | 50-1000 |

Although using a single concentrate source 104 in the system requires a fixed ratio of solutes in the generated peritoneal dialysate, a single concentrate source 104 provides certain advantages. Storage requirements are decreased, as only a single concentrate solution needs to be stored for a given dialysate prescription. There is also a lower risk of patient error in adding solutes to the dialysate in the proper amounts. A single concentrate source 104 also requires less supplies, less pumps, and less hardware. Further, because fewer containers are needed, the containers are easier to manage, clean, and disinfect. One of skill in the art will understand that a higher concentration of solutes in the concentrate source 104 will allow minimization of the container size and maximization of the source water used in PD solution preparation, lowering costs. The limiting factor is mutual solubility of the components, which is generally limited by glucose or icodextrin solubility. The flow rate for the source water can be optimized to adjust the time required to prepare the solution. In the case of on-demand dialysate preparation, a high flow rate is desired to minimize the time needed to prepare the solution. The flow rate limit will be controlled by the metering accuracy of the concentrate pump **105** at the rate required to match the water feed. With a single concentrate source **104,** about 150 ml/exchange can be needed, which corresponds to about 600 ml/day or 4.2 L/week. The concentrate source **104** can be sized depending on the needs of the user, with a larger concentrate source requiring less frequent refilling.

The system can also include an additional waste reservoir (not shown in FIG. 1) to collect any waste fluid generated by the water purification module **103** or other components. Alternatively, waste reservoir **121** can also be used to collect any waste fluid generated by the water purification module **103** or other components. The waste reservoir collects effluent generated during disinfection and/or effluent generated by the purification modules, such as a reverse osmosis system.

In any embodiment of the first or second aspects of the invention, the peritoneal dialysate generation flow path **101** and integrated cycler **110** can be disinfected with a disinfection solution through on-board disinfection. The peritoneal dialysate generation flow path **101** and integrated cycler **110** can be configured to form a loop by connecting the portion of the peritoneal dialysate generation flow path **101** that connects to water tank **102** or the direct connection **112** to a water source to the infusion line **124.** The disinfection solution can be introduced into the peritoneal dialysate generation flow path **101** and recirculated through the fluid lines by system pumps **108** and **119.** Alternatively, the peritoneal dialysate generation flow path **101** and integrated cycler **110** can be disinfected separately after disconnection of the integrated cycler **110** from the peritoneal dialysate generation flow path **101.** The disinfection solution can be a citric acid solution, a peracetic acid solution, a bleach solution, or any other disinfection solution known in the art. Disinfectant can be circulated through the flow loop and heated. The disinfectant can be heated to any temperature capable of disinfecting the system, including temperatures of at least 90 °C or greater. The disinfectant can be introduced to the flow loop and recirculated at elevated temperatures to ensure complete disinfection.

In any embodiment of the first or second aspects of the invention, solutes can be added to the peritoneal dialysate generation flow path **201** from two or more separate concentrate sources, as shown in FIG. 2. The peritoneal dialysate generation flow path **201** can be fluidly connected to a water source and a water purification module upstream of the concentrate sources **202-206,** and fluidly connected to a sterilization module, an integrated cycler, and optionally a dialysate container downstream of the concentrate sources **202-206,** as illustrated in FIG. 1. For clarity, these components have been omitted from FIG. 2.

As illustrated in FIG. 2, the concentrate sources **202-206** can include one or more ion concentrate sources, such as sodium chloride source **202** containing sodium chloride to be added in a controlled addition to the peritoneal dialysate generation flow path **201** by concentrate pump **207** through valve **212,** sodium lactate source **203** containing sodium lactate to be added in a controlled addition to the peritoneal dialysate generation flow path **201** by concentrate pump **208** through valve **213,** magnesium chloride source **204** containing magnesium chloride to be added in a controlled addition to the peritoneal dialysate generation flow path **201** by concentrate pump **209** through valve **214,** and calcium chloride source **205** containing calcium chloride to be added in a controlled addition to the peritoneal dialysate generation flow path **201** by concentrate pump **210** through valve **215.** One of skill in the art will understand that other ions can be used in formulation of peritoneal dialysate, and each can be contained in a separate ion concentrate source or combined into one or more combined ion concentrate sources. The concentrate source also includes at least two osmotic agent sources. One of these may be dextrose source **206** containing dextrose to be added to the peritoneal dialysate generation flow path **201** by concentrate pump **211** through valve **216.** Any of the concentrate pumps can include flow meters to control the addition of the solutes. A glucose source can be used in addition to, or in place of, dextrose source **206.** An icodextrin source is also used. Multiple osmotic agents can be added to the peritoneal dialysate generation flow path **201** from one or more osmotic agent sources. One of skill in the art will understand other solutes can be used alternatively to, or in addition to, the solutes illustrated in FIG. 2. Any set of solutes used for peritoneal dialysate is within the scope of the invention. A control system in electronic communication with each of the concentrate pumps can control the movement of fluid from the concentrate sources to the peritoneal dialysate generation flow path **201.** The amount of each of the concentrates moved into the peritoneal dialysate generation flow path **201** can be controlled to result in peritoneal dialysate having a prescribed solute concentration, as determined by a doctor or health care provider. The valves **212-216** can optionally be replaced with hose T junctions with additional components for preventing backflow into the concentrate source line if that particular line is not being used. Optional sensors **217, 218, 219,** and **220** ensure the solute concentration in the dialysate is at the correct level after each addition. The sensors **217-220** can be any type of sensor appropriate to confirm delivery of the concentrate, such as conductivity sensors. Optional pH sensor **221** ensures the pH is a proper level after addition of sodium lactate or other buffer. Optional refractive index meter **222** ensures the dextrose concentration in the dialysate is at the prescribed level. An additional sensor can be included upstream of sodium chloride source **202** for sensing the conductivity of the water prior to addition of concentrates. One of skill in the art will understand that additional sensor arrangements can be used in the described system. Any number of sensors can be included to monitor the peritoneal dialysate concentration, including 1, 2, 3, 4, 5, 6, 7, or more sensors. The concentrate sources can contain the solutes in either solid, powdered, or solution form. A solid or powdered source of solutes can be dissolved by the system by drawing fluid from the peritoneal dialysate generation flow path **201** into the concentrate source to generate a solution with a known concentration, such as a saturated solution of the solutes. The resulting solution is added to the peritoneal dialysate generation flow path as explained.

Although shown as a refractive index meter **222** in FIG. 2, one of skill in the art will understand that alternative methods of measuring the osmotic agent concentration can be used. In any embodiment, enzyme-based sensors can detect the concentration of the osmotic agent in the dialysate. Enzyme based sensors use an enzyme capable of oxidizing the osmotic agent, such as glucose or dextrose. The enzyme is immobilized on an electrode and covered in a membrane through which the osmotic agent can pass. The electrode is used to electrochemically measure the change in either the oxidant, such as oxygen, or the product of glucose oxidation, such as hydrogen peroxide. Alternatively, electron transfer between the electrode and the enzyme can be detected with mediators, such as ferrocene to facilitate electron transfer. The osmotic agents can alternatively be detected through pulsed amperometric detection (PAD). PAD can detect glucose by applying a positive potential to a sample, resulting in oxidation of the glucose. The oxidation products are adsorbed onto the electrode and then desorbed by applying a more positive potential. Applying the more positive potential results in formation of an oxide layer on the electrode leading to passivation of the electrode surface. The catalytic activity of the electrode is then restored by application of a more negative potential, resulting in dissolution of the oxide layer.

Although illustrated as a single concentrate source in FIG. 1, and five separate concentrate sources in FIG. 2, one of skill in the art will understand that any number of concentrate sources can generate the peritoneal dialysate, including 1, 2, 3, 4, 5, 6, 7, or more concentrate sources. The concentrate sources will have multiple osmotic agent sources and one or more ion concentrate sources. Any two or more of the separate concentrate sources illustrated in FIG. 2 can be combined into a single solute source, such as by combining all or some of the ion concentrate sources into a single ion concentrate source where the mixed contents do not cause precipitation of the mixed concentrates.

Although each concentrate source is illustrated in FIG. 2 with a separate concentrate pump and fluid line, one of skill in the art will understand that more than one concentrate source can use a single pump and fluid line, with valves arranged thereon for controlled addition to the peritoneal dialysate generation flow path **201.**

The concentrate sources **202-206** can be single use concentrate sources or disposable concentrate sources. The disposable concentrate sources are used in a single peritoneal dialysate generation process and then disposed. Multiple use concentrate sources are used repeatedly, and refilled as necessary with the solute.

Table 6 provides exemplary, non-limiting, ranges of solutes that can be added to the peritoneal dialysate using a separate osmotic agent source, glucose in Table 6, and a separate ion concentrate source containing sodium chloride, sodium lactate, magnesium chloride, calcium chloride and sodium bicarbonate. Because the glucose is added separately from the ion concentrates, the ratio of glucose to the other solutes can be varied depending on the needs of the patient.

**Table 6**

| Exemplary ranges of solutes in a two-concentrate source system | | | |
|---|---|---|---|
| **Component** | **Concentration (g/l)** | **Solution volume (ml/L)** | **Dialysate composition** |
| **Part A** | | | |
| Glucose | 850 | 6-53 | 0.55-4.5 g/dL |

| **Part B** | | | |
|---|---|---|---|
| NaCl | 269 | 20 | 92 mmol/L |
| Sodium Lactate | 84 | 20 | 15 mmol/L |
| MgCl₂.6H₂O | 5 | 20 | 0.5 mmol/L |
| CaCl₂.2H₂O | 18 | 20 | 2.5 mmol/L |
| NaHCO₃ | 105 | 20 | 25 mmol/L |
| Water | | 927-979 | 56.10 |

By using multiple concentrate sources, greater individualization and therapy customization can be achieved for each patient. With a single concentrate source, all solutes in the generated peritoneal dialysate must be present in a fixed ratio. By using more than one concentrate source, the ratio of solutes used in the peritoneal dialysate can be altered as the concentration of each of the osmotic agent and ion solutes can be individually controlled. For example, as illustrated by Table 6, with a single ion concentrate source and a single osmotic agent source, peritoneal dialysate with greater or less osmotic agent per concentration of ions can be generated, providing the ability to adjust the tonicity of the peritoneal dialysate solution independently of the electrolyte composition to meet the UF needs of any patient with a single set of solutions and allowing greater control over ultrafiltration. The ultrafiltration rate that results from using the peritoneal dialysate solutions can be altered by altering the concentration of the osmotic agent independently of the ionic solutes, or by changing the osmotic agent used. For example, typical ultrafiltration volumes using dextrose as the osmotic agent vary with the dextrose concentration of the peritoneal dialysate. With a 1.5% dextrose solution, the typical ultrafiltration volume is about 150 mL. With a 2.5 % dextrose solution, the typical ultrafiltration volume is about 250 mL. With a 4.25% dextrose solution, the typical ultrafiltration volume can exceed 600 mL. For a single exchange using separate concentrate sources for the ion concentrates and the osmotic agent, about 50 mL of the ion concentrate and 150 mL of the osmotic agent may be needed, corresponding to about 200 ml/day or 1.4 L/week of the ion concentrate and 600 ml/day or 4.2 L/week of the osmotic agent.

Because the system is not limited to discrete glucose or other osmotic agent concentrations like known commercial solutions; the system can customize the peritoneal dialysate solutions to meet the ultrafiltration needs of patient as determined by a healthcare provider. As illustrated in Table 6, the glucose level in the peritoneal dialysate solution can be varied from 0.55 g/dL to 4.5 g/dL, while maintaining the electrolytes and buffer components constant, allowing the system to cover the range of glucose formulations currently offered commercially using a single Part A and Part B composition.

In embodiments two osmotic agent sources are used; a glucose/dextrose source and an icodextrin source. With two osmotic agent sources, one could use dextrose during the daytime exchanges for CAPD and icodextrin during the night dwell to take advantage of the higher UF removal from icodextrin. Conversely, dextrose could be used during the night dwell and icodextrin for the extended daytime dwell in APD systems.

By using separate concentrate sources for each solute, complete individualization of the concentrations and ratios of solutes in the peritoneal dialysate can be achieved. Table 7 provides exemplary ranges of solutes that can be used in peritoneal dialysate as made by a system with each solute in a separate concentrate source. An advantage of using separate concentrate sources for each solute is that virtually any peritoneal dialysate solution composition can be prepared from a single set of component formulations. A system with separate concentrate sources for each solute is useful for patients whose prescriptions change periodically due to diet or other factors. Such patients would need to store multiple formulations if using only one or two concentrate sources, and the risk of errors would be increased.

**Table 7**

| Exemplary dialysate composition from a multi-source system | | | |
|---|---|---|---|
| **Component** | **Concentration (g/l)** | **Solution volume (ml/L)** | **Dialysate composition** |
| Part A: Glucose | 850 | 6 - 53 | 0.55-4.5 g/dL |
| Part B: NaCl | 320 | 15-18 | 132-134 mmol/L |
| Part C: Na Lactate | 1000 | 2-4 | 15-40 mmol/L |
| Part D: MgCl₂.6H₂O | 500 | 0.2-0.4 | 0.5 -1.0 mmol/L |
| Part E: CaCl2.2H2O | 700 | 0.5-1.0 | 2.5-3.5 mmol/L |
| Part F: NaHCO3 | 85 | 0-34 | 0-34 mmol/L |
| Part G: Icodextrin | 1000 | 0-75 | 0-7.5 g/dL |
| Water | | 820-971 | |

In any embodiment of the first or second aspects of the invention, the one or more concentrate sources can be detachable from the rest of the system for sterilization. The concentrate sources can also be sterilized each time the concentrate sources are filled with new concentrate solutions. Further, the concentrate sources can be sterilized after a set number of uses, or after a set period of time. Moreover, the concentrate sources and the rest of the peritoneal dialysate generation system can be sterilized without any of the components by passing a disinfection solution, such as a citric acid, peracetic acid, or bleach solution, through all of the lines and containers of the system.

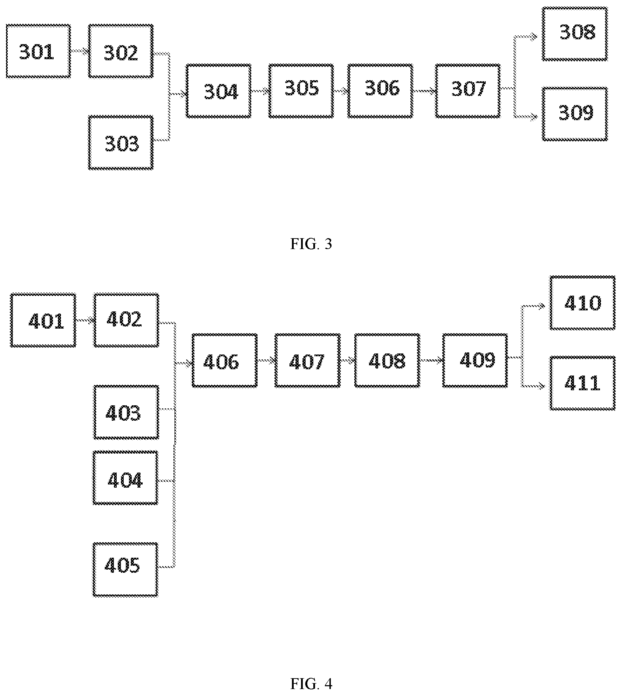

FIG. 3 illustrates an overview of generating peritoneal dialysate. This example does not form part of the claimed invention. Water from a water source **301** can be purified by a water purification module **302,** as explained. Concentrates from a single concentrate source **303,** which can contain both ion concentrates and one or more osmotic agents, can be added to the purified water to generate a non-sterile peritoneal dialysate solution **304.** The non-sterile peritoneal dialysate solution **304** is sterilized by a sterilization module **305,** which may include an ultrafilter (not shown). As explained, the peritoneal dialysate can be further purified by additional components in the sterilization module **306,** such as by ultrafiltration with a second ultrafilter, by a microbial filter, or by a UV light source, to generate a sterilized peritoneal dialysate **307.** The sterilized peritoneal dialysate **307** can be stored or used by any method described herein, including by immediately infusing the peritoneal dialysate into a patient **308,** or dispensing the peritoneal dialysate into a dialysate container for later use in peritoneal dialysis **309,** as illustrated in FIG. 1.

FIG. 4 illustrates an overview of generating peritoneal dialysate with multiple concentrate sources. Water from a water source **401** can be purified by a water purification module **402,** as explained. Concentrates from an ion concentrate source **403,** which can contain sodium, magnesium, calcium, and bicarbonate, as well as any other ions to be used in peritoneal dialysis, can be added to the purified fluid. An osmotic agent, such as dextrose, can be added from a first osmotic agent concentrate source **404.** A second osmotic agent, icodextrin, is be added from a second osmotic agent concentrate source **405.** As illustrated in FIG. 2, any number of concentrate sources can be used for further individualization of the peritoneal dialysate, including separate sources for each of the ions used. After addition of the ion and osmotic agent concentrates, the fluid contains all necessary components for use in peritoneal dialysis as non-sterilized peritoneal dialysate **406.** The non-sterile peritoneal dialysate **406** can be sterilized by a sterilization module **407,** which can include an ultrafilter or other sterilization components. The peritoneal dialysate can be further sterilized by the sterilization module **408,** either by ultrafiltration with a second ultrafilter, a microbial filter, or further sterilized with a UV light source, to generate a sterilized peritoneal dialysate **409.** The sterilized peritoneal dialysate 409 can be stored or used by any method described herein, including by immediately infusing the peritoneal dialysate into a patient **410,** or dispensing the peritoneal dialysate into a dialysate container for later use in peritoneal dialysis **411,** as illustrated in FIG. 1.

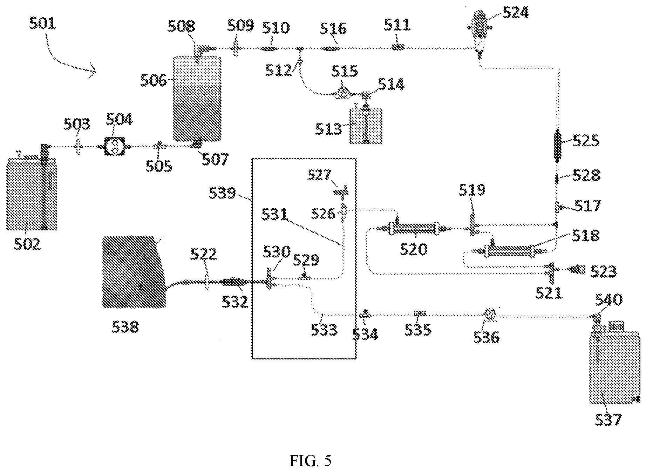

FIG. 5 illustrates an alternative peritoneal dialysate generation flow path **501** with an integrated cycler **539.** Water from a water source **502** can be pumped through filter **503** by system pump **504.** The filter **503** can remove any particulate matter from the water prior to entering the peritoneal dialysate generation flow path **501.** The water is then pumped through a water purification module, illustrated as a sorbent cartridge **506** in FIG. 5. As described, the water purification module can alternatively or additionally include activated carbon, a reverse osmosis module, a carbon filter, an ion exchange resin, and/or a nanofilter. The water enters the sorbent cartridge **506** through sorbent cartridge inlet **507** and exits through sorbent cartridge outlet **508.** Pressure sensor **505** measures the pressure across sorbent cartridge **506.** Filter **509** removes any particulate matter in the fluid after exiting sorbent cartridge **506.** A conductivity sensor **510** determines the conductivity of the fluid exiting sorbent cartridge **506** to ensure the water has been purified. To generate the peritoneal dialysate, concentrates are added from concentrate sources, such as **513** through concentrate connector **514** by concentrate pump **515.** Although shown as a single concentrate source **513** in FIG. 5, concentrates are added from any at least two separate concentrate sources. Concentrate filter **512** removes any particulate matter from the concentrate before entering the peritoneal dialysate generation flow path **501.** A conductivity sensor **516** determines the conductivity of the generated peritoneal dialysate after addition of the concentrates to ensure the peritoneal dialysate has the correct solute concentrations. Flow sensor **511** determines the flow rate of the fluid after addition of the concentrates. pH sensor **524** determines the pH of the peritoneal dialysate to ensure the peritoneal dialysate has a proper pH. The peritoneal dialysate can be heated to a desired temperature by heater **525.** Temperature sensor **528** ensures the peritoneal dialysate is heated to an appropriate temperature before infusion into the patient **538.** The heater **525** can be placed at any location in the flow path prior to delivery to the patient **538.** In any embodiment, the heater **525** can be located after the exit of the sterilization module, particularly if fluid is stored prior to passing through the sterilization module.

As described, the peritoneal dialysate is sterilized by pumping the peritoneal dialysate through a sterilization module, which can include first ultrafilter **518,** and optionally a second ultrafilter **520** and/or an optional UV light source (not shown). Pressure sensor **517** measures the fluid pressure prior to the fluid entering the sterilization module, shown as ultrafilters **518** and **520,** and is used in the control circuit to control the pressure. The fluid passes through first ultrafilter **518,** through valve **519,** and then through second ultrafilter **520.** Connector **523,** three way valve **521,** and valve **519** allow backflushing and disinfection of the ultrafilters **518** and **520.** The fluid is then pumped into the integrated cycler **539** for use in peritoneal dialysis. As described, the system can include a dialysate container (not shown) for storage of the generated peritoneal dialysate until used by the patient **538** at any location, including upstream or downstream of the sterilization module.

The integrated cycler **539** includes an infusion line **531** and a drain line **533.** Bubble trap **526** traps air bubbles present in the heated dialysate. The air is vented from the system through bubble trap valve **527.** Pressure sensor **529** ensures the pressure of the fluid is within a predetermined range. The infusion line **531** is connected to a three-way valve **530,** which controls fluid movement between the infusion line **531,** the patient **538,** and the drain line **533.** The three way valve **530** is connected through connector **532** to a catheter inserted into the peritoneal cavity of the patient **538.** A filter **522** can be included between the three-way valve **530** and the catheter for additional cleaning of the peritoneal dialysate prior to entering a patient **538.** In any embodiment, the filter **522** can be a disposable filter. The peritoneal dialysate is infused into the patient **538** and held for a dwell period. After the dwell period, the fluid is pumped out of the peritoneal cavity of the patient **538** by drain pump **536.** The three-way valve **530** is switched to direct fluid into the drain line **533.** Pressure sensor **534** measures the pressure of fluid in the drain line **531** to ensure proper drainage. Flow meter **535** measures the flow rate and volume of fluid removed from the patient **538.** The drain line **531** is connected to a drain or waste reservoir **537** through connector **540** for collection and disposal of the used peritoneal dialysate.

For automated disinfection of the system, connector **540** can be connected to connector **523** to form a flow loop. Disinfectant can be circulated through the flow loop and heated. The disinfectant can be heated to any temperature capable of disinfecting the system, including temperatures of at least 90 °C or greater. The disinfectant can be introduced to the flow loop and recirculated at elevated temperatures to ensure complete disinfection. The disinfectant used can be any suitable disinfectant known in the art, including peracetic acid, citric acid, or bleach. The connectors and components of the system can be gamma and autoclave compatible to resist the high temperatures used during disinfection. The system can be primed by introducing a priming fluid to the peritoneal dialysate generation flow path **501** and integrated cycler **539.**

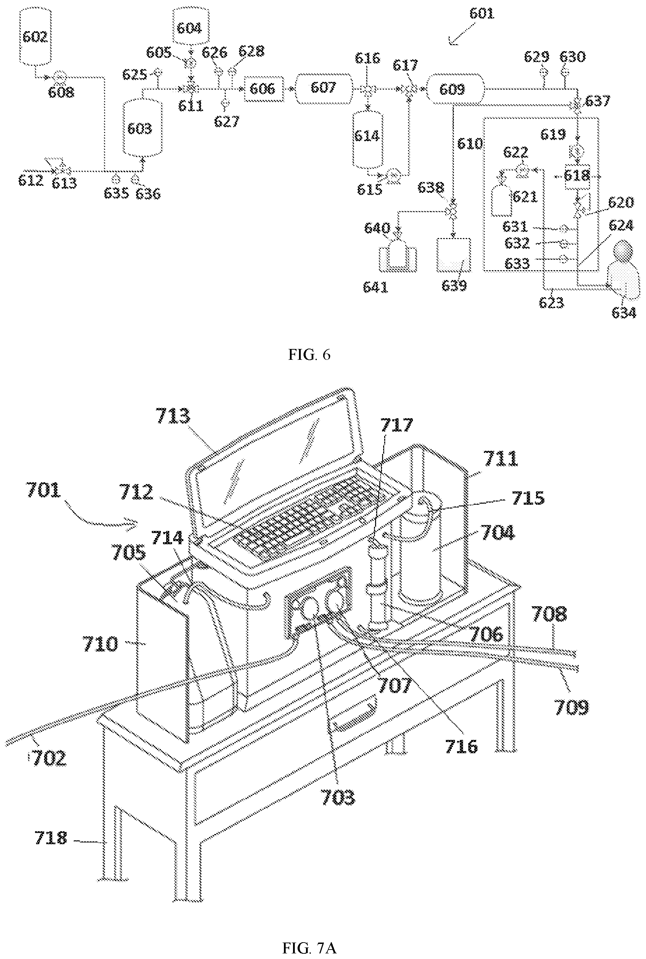

FIG. 6 illustrates a system which does not form part of the claimed invention.. Fluid from a water source, such as water tank **602,** can be pumped into the peritoneal dialysate generation flow path **601.** Additionally, or as an alternative to a water tank **602,** the system can use a direct connection to a water source **612.** System pump **608** can control the movement of fluid through the peritoneal dialysate generation flow path **601.** If a direct connection to a water source **612** is used, a pressure regulator **613** can ensure that an incoming water pressure is within a predetermined range. The system pumps the fluid from water source **608 or 612** through a water purification module **603** to remove chemical contaminants in the fluid in preparation for creating dialysate.

After the fluid passes through the water purification module **603,** the fluid is pumped to a concentrate source **604,** where necessary components for carrying out peritoneal dialysis can be added from the concentrate source **604.** The concentrates in the concentrate source **604** are utilized to create a peritoneal dialysis fluid that matches a dialysis prescription. Concentrate pump **605** and concentrate valve **611** can control the movement of concentrates from the concentrate source **604** to the peritoneal dialysate generation flow path **601** in a controlled addition. Alternatively, concentrate valve **611** can be a hose T or backflow restricting hose T. The concentrates added from the concentrate source **604** to the peritoneal dialysate generation flow path **601** can include components required for use in peritoneal dialysate. Upon addition of solutes from the concentrate source **604,** the fluid in the peritoneal dialysate generation flow path **601** can contain all the necessary solutes for peritoneal dialysis. The peritoneal dialysate should reach a level of sterility for peritoneal dialysis, as described. As shown in FIG. 6, the sterilization module can include one or more of a first ultrafilter **607,** a second ultrafilter **609,** and a UV light source **606.**

The generated peritoneal dialysate can be pumped directly to an integrated cycler **610** for immediate infusion into a patient **634.** Alternatively, the dialysate can be pumped to an optional dialysate container **614** as a pre-prepared bolus of solution for storage until ready for use by a patient **634.** Valve **616** can control the movement of fluid to either the dialysate container **614.** Stored dialysate in dialysate container **614** can be pumped as needed to back into the peritoneal dialysate generation flow path **601** by pump **615** through valve **617.** The dialysate container **614** can store enough peritoneal dialysate for a single infusion of peritoneal dialysate into the patient **634,** or enough peritoneal dialysate for multiple or continuous infusions into one or multiple patients.

The generated peritoneal dialysate can be pumped to valve **637.** Valve **637** can control movement of the peritoneal dialysate to any of three options. First, the peritoneal dialysate can be pumped to integrated cycler **610,** second diverted for use with a non-integrated external cycler **639,** or third diverted to a dialysate container **640.** All three options can be performed contemporaneously or selectively. If diverted to the non-integrated external cycler **639,** the peritoneal dialysate can be pumped via valve **638.** Valve **638** can control the movement of the peritoneal dialysate through either a direct connection to an external cycler **639** or to a dialysate container **640.** Alternative valve and pump configurations for performing the same functions are contemplated by the present invention. For example, the direct connection to an external cycler **639** can use any type of connector known in the art. The connectors can be single-use or reusable connectors and should provide for sterile transfer of fluids. The connectors should preferably be closed connectors, to avoid contact between the fluids and the external environment. A non-limiting example of a connector that can be used for a direct connection to an external cycler is the INTACT^{®} connectors provided by Medinstill Development LLC, Delaware, US. The dialysate container **640** can be heated with an optional heater **641** and then used in peritoneal dialysis. The connectors to the dialysate container **640** can be any type of connector known in the art. The connectors can be single use or disposable connectors that provide transfer of sterile fluids. A non-limiting example of connectors that can be used with the described system is the Lynx^{®}-Millipore connectors available from Merck KGaA, Darmstadt, Germany.

The integrated cycler **610** can include a metering pump **619** for metering peritoneal dialysate into the peritoneal cavity of the patient **634.** A heater **618** heats the peritoneal dialysate to a desired temperature prior to infusion into the patient **634.** A pressure regulator **620** ensures the peritoneal dialysate pressure is within a predetermined range safe for infusion into the patient **634.** The metering pump **619** can use any safe pressure for infusing fluid into the patient **634.** Generally, the pump pressures are on average set at ±10.3 kPa or 77.6 mmHg. If there is no fluid flow, the maximum pressure can increase to ±15.2 kPa or 113.8 mmHg for a short period, such as less than 10 seconds. The peritoneal dialysate is infused into the peritoneal cavity of the patient **634** through infusion line **624.** After a dwell period, the peritoneal dialysate is drained from the patient **634** through drain line **623.** Pump **622** provides a driving force for removing the peritoneal dialysate from the patient **634.** An optional waste reservoir **621** can be included to store the used peritoneal dialysate for disposal. Alternatively, the drain line **623** can be directly connected to a drain for direct disposal. The waste reservoir **621** can be any size, including between 12 and 20 L. For patients requiring a higher drainage, a drain manifold can be included for connecting multiple waste reservoirs.

Various sensors positioned in the peritoneal dialysate generation and infusion system ensure that the generated fluid is within predetermined parameters. Flow meter **635** ensures the incoming water is at a correct flow rate, while pressure sensor **636** ensures the incoming water is at an appropriate pressure. Conductivity sensor **625** is used to ensure that the water exiting water purification module **603** has been purified to a level safe for use in peritoneal dialysis. Conductivity sensor **626** ensures the conductivity of the dialysate after the addition of concentrates from concentrate source **604** is within a predetermined range. Refractive index sensor **627** insures that the concentration of the osmotic agents is within a predetermined range. pH sensor **628** ensures the pH of the peritoneal dialysate is within a predetermined range. After passing through the sterilization module including second ultrafilter **609,** pH sensor **629** and conductivity sensor **630** are used to ensure that no changes in the pH or conductivity have occurred during purification or storage of the dialysate in dialysate container **614.** The integrated cycler **610** has flow meter **631,** pressure sensor **632** and temperature sensor **633** to ensure that the dialysate being infused into the patient **634** is within a proper flow rate, pressure, and temperature range.

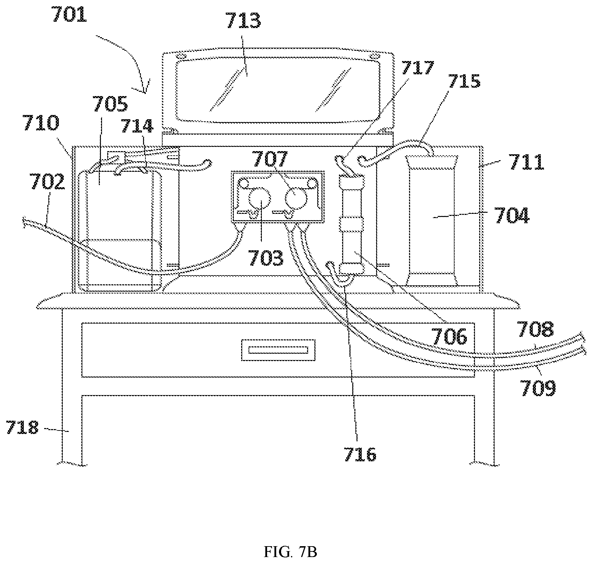

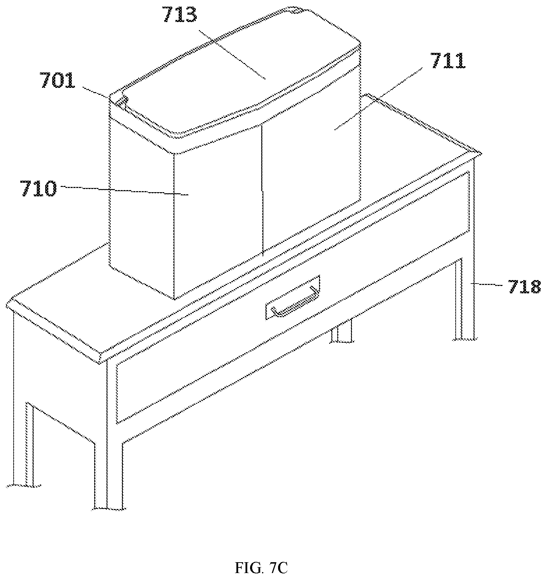

FIG.'s 7A-7B illustrate examples of a peritoneal dialysate generation cabinet **701** which do not form part of the claimed invention. FIG. 7A illustrates a perspective view of the peritoneal dialysate generation cabinet **701,** while FIG. 7B illustrates a front view of the peritoneal dialysate generation cabinet **701.** A fluid line **702** can connect a water source (not shown) to the peritoneal dialysate generation cabinet **701.** System pump **707** provides a driving force for the movement of fluid throughout the peritoneal dialysate generation flow path as described with respect to FIG.'s 1 and 5-6. The water is pumped through the peritoneal dialysate generation cabinet **701** to a water purification module, shown as sorbent cartridge **704** in FIG.'s 7A-B. The water enters the sorbent cartridge **704** through tubing (not shown) connected to the bottom of the sorbent cartridge through the base of the peritoneal dialysate generation cabinet **701,** and exits through tubing **715** at a top of the sorbent cartridge **704.** Concentrates from concentrate source **705** are added to the fluid through tubing **714** as described to generate non-sterilized peritoneal dialysate. A concentrate pump (not shown) can provide a driving force to move fluid from the concentrate source **705** into the peritoneal dialysate generation flow path inside of the cabinet. The generated peritoneal dialysate is then pumped through a sterilization module, shown as ultrafilter **706,** for sterilization. The peritoneal dialysate enters the ultrafilter **706** through tubing **716** in a base of the ultrafilter **706** and exits through tubing **717** at a top of the ultrafilter **706.** A second ultrafilter, microbial filter and/or UV light source (not shown in FIG. 7) can also be included. The peritoneal dialysate is then heated by a heater (not shown in FIG. 7) and pumped into the peritoneal cavity of a patient through infusion line **708** by metering pump **703.** After a dwell period, the peritoneal dialysate is drained from the patient through drain line **709.** As described, the peritoneal dialysate generation flow path can include various sensors for detection of conductivity, pH, refractive index, temperature, or other dialysate parameters. The sensors can be included either inside or outside of the body of the peritoneal dialysate generation cabinet **701.** The fluid lines and valves connecting the components of the peritoneal dialysate generation flow path can likewise be positioned inside of the cabinet body. As described, peritoneal dialysate generation cabinet **701** can have a graphical user interface including screen **713** and keyboard **712.** Messages from the control system to the user, or from the user to the control system, can be generated and read through the graphical user interface. The user can direct the generation of peritoneal dialysate through keyboard **712,** and can receive messages from the system through screen **713.** The system can generate alerts to the user, including any problems detected by any of the sensors, as well as the progress of peritoneal dialysate generation. Any type of user interface can be used in place of the keyboard **712** and screen **713** in FIG.'s 7A-B. Alternatively, other interfaces can be included, such as lights, dials, buttons, switches or the like. In any embodiment, a single button can be used for directing the generation of peritoneal dialysate in place of the keyboard **712.** In any embodiment, either keyboard **712** or screen **713** can be used alone, as with a single touch screen for both data entry and display to enable simple operation.

FIG. 7C illustrates the peritoneal dialysate generation cabinet **701** after being closed. If not in use, the concentrate source **705,** the sorbent cartridge **704,** and the tubing to and from the patient can be removed, and the doors **710** and **711** of the peritoneal dialysate generation cabinet **701** can be closed to minimize storage space. Additionally, the screen **713** of the graphical user interface illustrated in FIG.'s 7A and 7B can be folded down into the top of the peritoneal dialysate generation cabinet **701,** further minimizing storage space. The doors **710** and **711** can be open and closed by any method known in the art, including magnets, handles, indentations, hooks, or any other method of opening and closing the doors **710** and **711.** The peritoneal dialysate generation cabinet **701** can have a small size and portability optimized for in-home or beside use. Although shown on table **718,** the peritoneal dialysate generation cabinet **701** can be used on any stable flat surface.

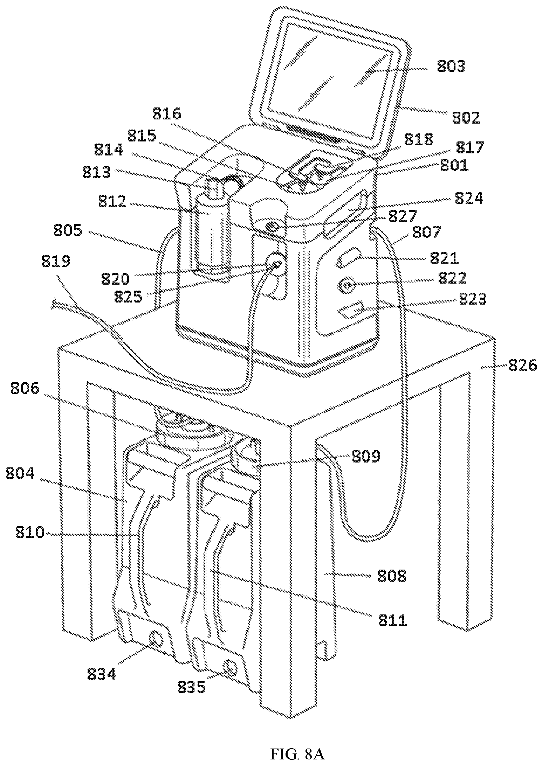

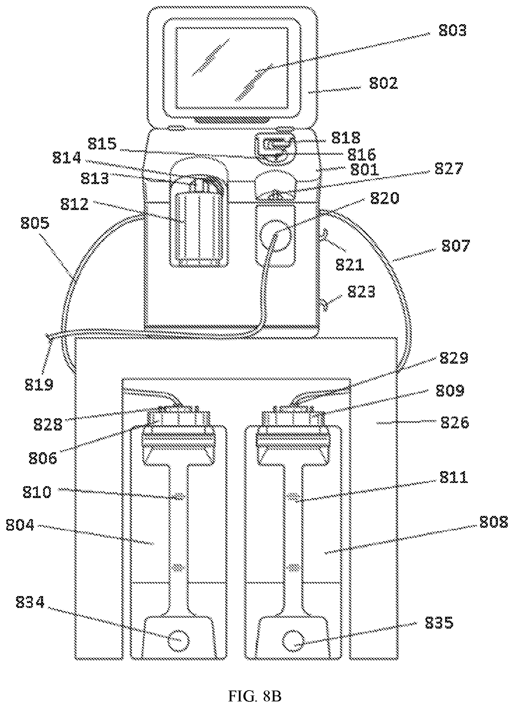

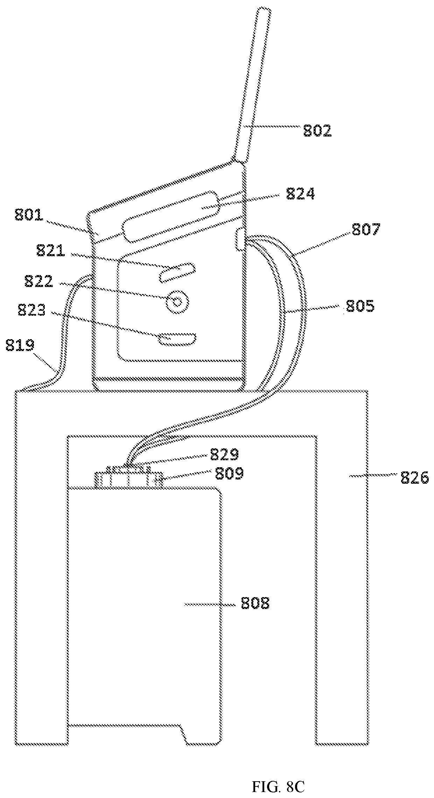

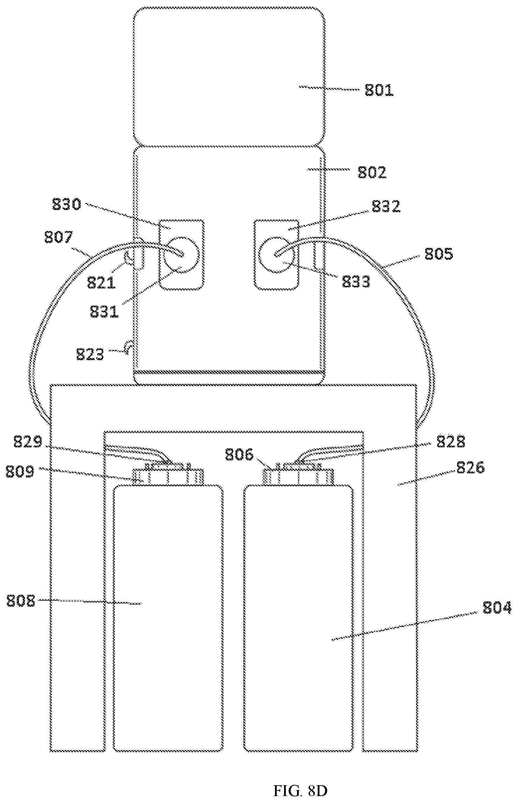

FIG.'s 8A-D a peritoneal dialysate generation system arranged as a peritoneal dialysate generation cabinet **801,** which does not form part of the claimed invention. FIG. 8A illustrates a perspective view of the peritoneal dialysate generation cabinet **801,** FIG. 8B illustrates a front view of the peritoneal dialysate generation cabinet **801,** FIG. 8C illustrates a side view of the peritoneal dialysate generation cabinet **801,** and FIG. 8D illustrates a back view of the peritoneal dialysate generation cabinet **801.**

A fluid line **805** can connect a water source **804** to the peritoneal dialysate generation cabinet **801.** The fluid line **805** can enter through a connector **828** in a top **806** of the water source **804.** The fluid line **805** connects to the peritoneal dialysate generation flow path as described with reference to FIG.'s 1 and 5-6 through a back of the peritoneal dialysate generation cabinet **801** through connector **832** having a fitting **833** for holding the fluid line **805,** as illustrated in FIG. 8D. Any of the fluid lines illustrated can be disconnected and removed from the system for cleaning and replacement. A pump (not shown) can provide a driving force for the movement of fluid throughout the peritoneal dialysate generation flow path if required. Water is pumped through the peritoneal dialysate generation cabinet **801** to a water purification module, shown as sorbent cartridge **812** in FIG.'s 8A-B. The water can enter the sorbent cartridge **812** through tubing (not shown) connected to the bottom of the sorbent cartridge **812** within the peritoneal dialysate generation cabinet **801.** The water exits the sorbent cartridge **812** through connector **813** and tubing **814.** An osmotic agent from osmotic agent source **815** and an ion concentrate from an ion concentrate source **817** are added to the fluid as described to generate non-sterilized peritoneal dialysate. The osmotic agent concentrate is added to the fluid through paddle connector **816.** The ion concentrate is added to the fluid through paddle connector **818.** A concentrate pump (not shown) can provide a driving force to move fluid from the concentrate sources into the peritoneal dialysate generation flow path inside of the peritoneal dialysate generation cabinet **801.** As described, the system can use a single ion concentrate source in place of the two sources shown in FIG.'s 8A-B, or more than two concentrate sources. The generated peritoneal dialysate can then be pumped through a sterilization module (not shown), such as an ultrafilter. A second ultrafilter and/or a UV light source can also be included. An integrated cycler (not shown in FIG.'s 8A-D) can then pump the dialysate into infusion line **819** through connector **820** and into the patient. Fitting **825** allows the infusion line **819** to be removed from the system for cleaning or replacement. Waste fluids can be pumped out of the system through waste line **807,** which connects to the peritoneal dialysate generation cabinet **801** through connector **830** having fitting **831.** A separate waste line for removing used dialysate from the patient (not shown in FIG.'s 8A-D) can also connect to the peritoneal dialysate generation cabinet **801** and connect to waste line **807.** The waste line **807** enters waste container **808** through a connector **829** in the top **809** of the waste container **808.** Handles **810** and **811** can be included on water source **804** and waste container **808** for easy movement and storage. Although the peritoneal dialysate generation cabinet **801** is illustrated on top of table **826** in FIG.'s 8A-D, the peritoneal dialysate generation cabinet **801** can be used on any stable flat surface.

As described, the peritoneal dialysate generation flow path can include various sensors for detection of conductivity, pH, refractive index, or other dialysate parameters. The sensors can be included either inside or outside of the body of the peritoneal dialysate generation cabinet **801.** The fluid lines and valves connecting the components of the peritoneal dialysate generation flow path can likewise be positioned inside of the cabinet body. As described, a top of the peritoneal dialysate generation cabinet **801** can have a graphical user interface **802** including screen **803.** Messages from the control system to the user, or from the user to the control system, can be generated and read through the graphical user interface **802.** The user can direct the generation of peritoneal dialysate through the graphical user interface **802,** and can receive messages from the system through screen **803.** The system can generate alerts to the user, including any problems detected by any of the sensors, as well as the progress of peritoneal dialysate generation. A handle **824** can be included for opening the peritoneal dialysate generation cabinet **801** to allow access to components on the inside of the cabinet. Handles **821** and **823** can be included to hold the fluid lines and power cord when not in use.

Disinfection connector **822** illustrated in FIG.'s 8A and 8C can be included for disinfection of the waste line **807.** During disinfection, the waste line **807** can be disconnected from waste container **808** and connected to disinfection connector **822.** Disinfectant solution from a disinfectant source (not shown in FIG.'s 8A-D) can then be circulated through the waste line **807** to disinfect the waste line **807.** Disinfection connector **827** can be included for disinfection of fluid line **805.** Fluid line **805** can be connected to disinfection connector **822** and disinfection solution can be circulated through the fluid line **805.** Drain **834** on water source **804** and drain **835** on waste container **808,** allow the water source **804** and waste container **808** to be drained without inverting the containers.

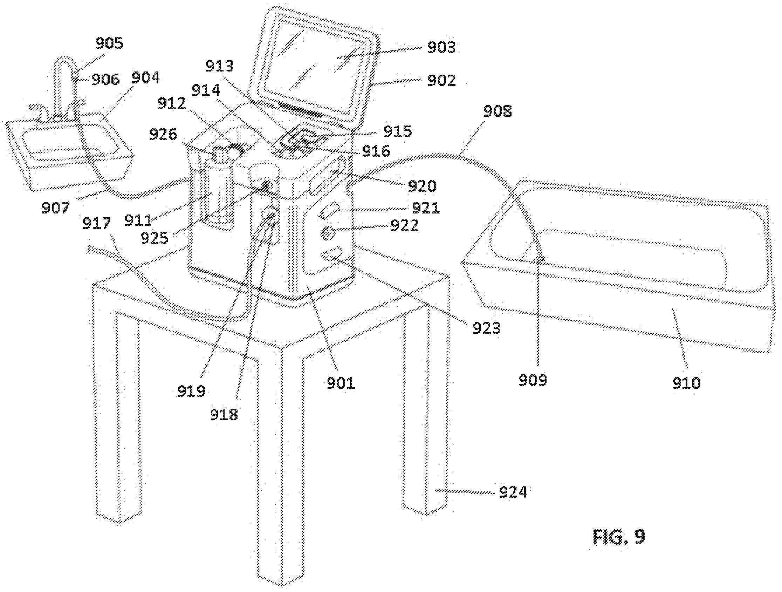

FIG. 9 illustrates a peritoneal dialysate generation cabinet **901** using a non-purified water source, faucet **905** in sink **904.** This example does not form part of the claimed invention. Although illustrated as faucet **905** and sink **904,** one of ordinary skill in the art will understand that any water source can be used. The ability to use municipal or other non-purified sources of water allow the peritoneal dialysate generation system to work at a patient's home without the need to store large amounts of purified water or dialysate. Fitting **906** connects the water line **907** to the faucet **905** or other water source, allowing the water line **907** to be connected or disconnected as necessary. A pump (not shown) can provide a driving force for the movement of fluid throughout the peritoneal dialysate generation flow path as described with respect to FIG.'s 1 and 5-6. The water is pumped through the peritoneal dialysate generation cabinet **901** to a water purification module, shown as sorbent cartridge **911** in FIG. 9. The water enters the sorbent cartridge **911** through tubing (not shown) connected to the bottom of the sorbent cartridge **911** within the peritoneal dialysate generation cabinet **901.** The water exits the sorbent cartridge **911** through connector **926** and tubing **912.** An osmotic agent from osmotic agent source **913** and an ion concentrate from an ion concentrate source **914** are added to the fluid as described to generate non-sterilized peritoneal dialysate. The osmotic agent concentrate is added to the fluid through paddle connector **916.** The ion concentrate is added to the fluid through paddle connector **915.** A concentrate pump (not shown) can provide a driving force to move fluid from the concentrate sources into the peritoneal dialysate generation flow path inside of the peritoneal dialysate generation cabinet **901.** As described, the system can use a single ion concentrate source in place of the two sources shown in FIG. 9, or more than two concentrate sources. The generated peritoneal dialysate can then be pumped through a sterilization module (not shown), such as an ultrafilter. A second ultrafilter and/or a UV light source can also be included. An integrated cycler (not shown in FIG. 9) can then pump the dialysate into infusion line **917** through connector **918** and into the patient. Fitting **919** allows the infusion line **917** to be removed from the system for cleaning or replacement. Waste fluids can be pumped out of the system through waste line **908,** which can connect to a drain **909** shown in bathtub **910.** A separate drain line (not shown) from the patient can be included to move used dialysate into the drain **909.** Although shown as a bathtub drain **909** in FIG. 9, the waste fluids can be conveyed to any type of drain, or alternatively to a waste container as illustrated in FIG.'s 8A-D. Although the peritoneal dialysate generation cabinet **901** is illustrated on top of table **924** in FIG. 9, the peritoneal dialysate generation cabinet **901** can be used on any stable flat surface. In certain embodiments, the peritoneal dialysate generation cabinet **901** and the patient can be in the same room as the water source and drain **909.** Alternatively, the patient and/or peritoneal dialysate generation cabinet **901** can be in a separate room, with tubing long enough to reach patient. For longer distances, the tubing should be strong enough to withstand the pressures necessary in pumping fluid over longer distances.

As described, a top of the peritoneal dialysate generation cabinet **901** can have a graphical user interface **902** including screen **903.** Messages from the control system to the user, or from the user to the control system, can be generated and read through the graphical user interface **902.** The user can direct the generation of peritoneal dialysate through the graphical user interface **902,** and can receive messages from the system through screen **903.** The system can generate alerts to the user, including any problems detected by any of the sensors, as well as the progress of peritoneal dialysate generation. A handle **920** can be included for opening the peritoneal dialysate generation cabinet **901** to allow access to components on the inside of the cabinet. Handles **921** and **923** can be included to hold the fluid lines and power cord when not in use.

Disinfection connector **922** can be included for disinfection of the waste line **908.** During disinfection, the waste line **908** can be disconnected from the drain **909** and connected to disinfection connector **922.** Disinfectant solution from a disinfectant source (not shown in FIG. 9) can then be circulated through the waste line **908** to disinfect the waste line **908.** Disinfection connector **925** can be included for disinfection of water line **907.** The water line **907** can be disconnected from faucet **905** and connected to disinfection connector **925.** Disinfectant solution can be circulate through the water line **907** for disinfection.

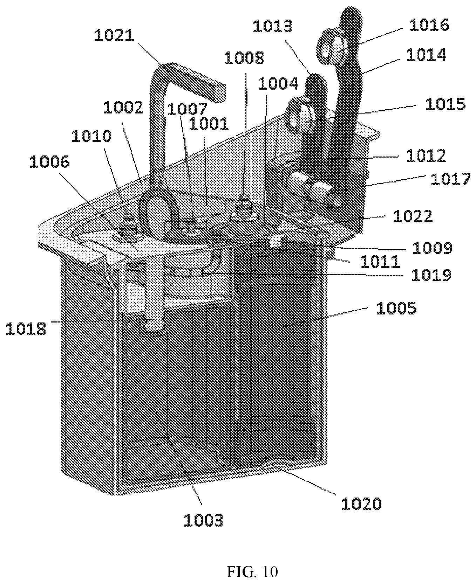

In any embodiment of the first or second aspects of the invention, the solute sources included in the dialysate generation module can be provided in a dialysis caddy. A dialysis caddy is a container adapted to contain one or more other containers, each having one or more solute sources. A dialysis caddy is shown in FIG. 10. This does not form part of the claimed invention. The dialysis caddy **1001** can contain some or all of the solute sources necessary for peritoneal dialysis. In any embodiment of the first or second aspects of the invention, the dialysis caddy **1001** can contain an ion concentrate source **1003,** osmotic agent source **1004,** and sodium chloride source **1005.** As explained, the ion concentrate source **1003** can contain any one or more of ion concentrates, such as magnesium chloride, calcium chloride or potassium chloride, or any other solutes used in peritoneal dialysis. Osmotic agent source **1004** can contains at least two osmotic agents: glucose/dextrose, and icodextrin. One of skill in the art will understand that any of the solutes can be contained in separate sources, and that the dialysis caddy **1001** can be adapted for any number of concentrate sources. In use, the dialysis caddy **1001** can be placed in a receiving slot of a dialysis system **1002.** As shown in FIG. 10, the dialysis caddy **1001** can be configured so that each of the ion concentrate source **1003,** osmotic agent source **1004,** and sodium chloride source **1005** are aligned with connectors for connection to the peritoneal dialysate generation flow path, such as the connectors on paddle assemblies **1013** and **1014.** In any embodiment of the first or second aspect of the invention, the dialysis caddy **1001** can also contain a disinfectant source **1006,** which may contain a disinfectant, such as citric acid. To disinfect the system, the dialysis caddy **1001** can be turned so container connectors **1010** and **1011** on the disinfectant source **1006** can connect to the connectors on paddle assemblies **1013** and **1014.**

If the dialysis caddy **1001** is configured to generate peritoneal dialysate, container connector **1007** on ion concentrate source **1003** and container connector **1008** on osmotic agent source **1004** can connect to caddy connectors **1015** on paddle assembly **1013** and caddy connector **1016** on paddle assembly **1014.** Container connector **1009** on sodium chloride source **1005** can also connect to a caddy connector (not shown in FIG. 10). The paddles can form a part of paddle assembly **1012.** To connect the sources to the paddles, the paddles can be rotated downward on hinge **1017** and the caddy connectors **1015** and **1016** can connect to ion concentrate source **1003** and osmotic agent source **1004** respectively. In any embodiment of the first or second aspects of the invention, as shown in FIG. 10, the dialysis caddy **1001** and the sources within the caddy have one or more fitting feature to ensure the sources are connected to the correct paddle. The fitting features can also have the additional benefit of ensuring a tight fit within the dialysis caddy **1001,** and resist inadvertent movement. The one or more fitting features can ensure each source occupies a unique position within the dialysis caddy **1001.** Moreover, in any embodiment, the interior of the dialysis caddy **1001** can itself be a shaped fitting feature so each source can only be placed within a specific position or receiving compartment within the dialysis caddy **1001.** In any embodiment of the first or second aspects of the invention, fitting features can be included on any connection surface of the caddy, where any source contacts the interior of the caddy. The shape of a caddy surface can include fitting feature protrusion **1020,** which is a protrusion on the base of the dialysis caddy **1001.** The base of sodium chloride source **1005** can be designed with a corresponding complementary indentation, such as a similarly sized recess, while the other sources lack the complimentary indentation. Sodium chloride source **1005** will be the only source that can properly fit into the position in the caddy above the fitting feature of protrusion **1020.** Similarly, fitting feature protrusion **1022** is a protrusion in the side of the dialysis caddy **1001** interior. The protrusion **1022** separates the sidewall of the dialysis caddy **1001** interior into two sections. Osmotic agent source **1004** can be the only source with the proper size, shape, or geometry to fit within one of the sections on the sidewall, whereas sodium chloride source **1005** can be the only source with the proper size, shape, or geometry to fit within the other section. Each concentrate source can be positioned in one particular location within the dialysis caddy **1001.** In any embodiment, the concentrate sources themselves can have fitting features that ensure the proper arrangement of the concentrate sources within the dialysis caddy **1001.** In FIG. 10 disinfectant (e.g. citric acid) source **1006** includes flange **1018.** Ion concentrate source **1003** has a corresponding slot. The disinfectant (e.g. citric acid) source **1006** can only be placed within the dialysis caddy **1001** at the precise position above ion concentrate source 1003. By sizing and shaping the interior of the cavity and the concentrate sources, the concentrate sources can only be placed within the dialysis caddy **1001** in a single arrangement. If the dialysis caddy **1001** is attached to the rest of the dialysis system **1002,** the concentrate sources and connectors line up with the proper paddles for connection to the dialysis system, ensuring the proper solutes from the concentrate sources enter the dialysate flow path at the correct locations. The alignment also ensures the proper pumps and valves are controlling the correct solute additions. In any embodiment of the first or second aspects of the invention, handle **1021** can be included for easy of carrying and removal of the dialysis caddy **1001** from the dialysis system **1002.** During use, fluid lines, such as line **1019** in disinfectant (*e.g*. citric acid) source **1006,** can move fluids from the concentrate sources into the paddles.

Alternatively, any method of loading the peritoneal dialysate concentrates can be included in the described systems. For example, the peritoneal dialysate concentrates can be added using a disposable cassette. The disposable cassette can be multi-use or single-use with disposal of the cassette after therapy.

The connectors can include connectors for connection to reservoirs, containers, or a tap or faucet. The connectors can be any type of connector that can form a seal with a container, tap, or faucet that serve as the fluid sources in the system. The connectors can be screw-type connectors that screw onto the containers, faucet or tap, snap-type connectors that snap onto the containers, faucet, or tap, or any other type of connector known in the art. O-rings or other sealing members can be included in the connectors to form a water-tight seal with the containers, faucet, or tap.

For connection to a tap or faucet, the connectors should be able to form a seal with standard at-home faucets. The connectors can include an adjustable bore, wherein the size of the opening of the connector for connection to the tap or faucet can be increased or decreased to adjust to different size faucets. Nuts, screws, or other tightenable components can be included on the sides of the connectors allowing a user to tighten the connector around the faucet or tap regardless of the circumference of the faucet or tap. An o-ring or other sealing member can be placed on the faucet or tap to increase the effectiveness of the seal formed with the connectors.

Alternatively, a fitting can be screwed onto, or otherwise affixed to the faucet with a male end of the fitting extending outwardly from the faucet. The male end of the fitting can be inserted into the water line, and secured with an adjustable bolt, wire, or other tightening mechanism to ensure a proper seal.

For connection to a drain as illustrated in FIG. 9, the tubing for carrying waste fluids can simply be placed into the drain, bathtub, or other receptacle containing a drain for disposal. Alternatively, the tubing can include a connector for forming a sealable connection to a drain, ensuring that all waste fluids are directed into the drain.

One skilled in the art will understand that various combinations and/or modifications and variations can be made in the described systems and methods depending upon the specific needs for operation.

## Claims

1. A system, comprising:
a water source (401);
a peritoneal dialysate generation flow path; wherein the peritoneal dialysate generation flow path is fluidly connectable to the water source (401);
one or more water purification modules (402) fluidly connectable to the peritoneal dialysate generation flow path;
a concentrate source fluidly connectable to the peritoneal dialysate generation flow path; the concentrate source containing one or more solutes and comprising multiple osmotic agent sources (404, 405) and one or more ion concentrate sources (403);
wherein the multiple osmotic agent sources comprise a first osmotic agent source (404) consisting essentially of glucose or dextrose and a second osmotic agent source (405) consisting essentially of icodextrin;
a sterilization module (407) fluidly connectable to the peritoneal dialysate generation flow path; and
an integrated cycler (539) fluidly connected to the peritoneal dialysate generation flow path,
and further comprising a concentrate pump (515) positioned between each of the multiple osmotic agent sources (404, 405) and the peritoneal dialysate generation flow path for controlled addition of fluid from each of the multiple osmotic agent sources (404, 405) to the peritoneal dialysate generation flow path.

2. The system of claim 1, further comprising one or more dialysate containers fluidly connectable to the peritoneal dialysate generation flow path downstream of the sterilization module (407).

3. The system of any preceding claim, wherein the ion concentrate source comprises one or more from the group consisting of sodium chloride, sodium lactate, magnesium chloride, calcium chloride, potassium chloride, and sodium bicarbonate.

4. The system of any preceding claim, further comprising a control system for controlling one or more pumps and valves to control movement of fluid through the system.

5. The system of claim 6, wherein the control system comprises a timer, and wherein the timer causes the control system to generate peritoneal dialysate at a predetermined time.

6. The system of claim 4 or claim 5, wherein the control system comprises a user interface (902), wherein the user interface (902) causes the control system to generate peritoneal dialysate at a selected time.

7. The system of any preceding claim, wherein the sterilization module (407) comprises one or more from the group consisting of one or more ultrafilters (518, 520), a UV light source, a heater, a flash pasteurization module, a microbial filter, and combinations thereof, preferably wherein the sterilization module (407) comprises the UV light source positioned downstream of the ultrafilter (518, 520).

8. The system of any preceding claim, wherein the water purification module (402) comprises one or more selected from the group consisting of a sorbent cartridge (506), activated carbon, a reverse osmosis module, a carbon filter, and a nanofilter.

9. The system of any preceding claim, wherein the integrated cycler (539) comprises a heater and a pump.

10. The system of any preceding claim, wherein the integrated cycler (539) further comprises at least one sensor selected from the group consisting of a flow meter, a pressure sensor, a conductivity sensor, and a temperature sensor.

11. The system of any preceding claim, wherein the integrated cycler (539) comprises an infusion line (531) and a drain line (533).

12. The system of claim 11, wherein the drain line (533) is fluidly connected to a waste reservoir (537).

13. The system of any preceding claim, wherein the integrated cycler (539) further comprises a filter (522) in the infusion line (531).

## Patentansprüche

1. System, umfassend:
eine Wasserquelle (401);
einen Peritonealdialysaterzeugungsströmungsweg; wobei der Peritonealdialysaterzeugungsströmungsweg mit der Wasserquelle (401) fluidisch verbindbar ist;
ein oder mehrere Wasserreinigungsmodule (402), die mit dem Peritonealdialysaterzeugungsströmungsweg fluidisch verbindbar sind;
eine Konzentratquelle, die mit dem Peritonealdialysaterzeugungsströmungsweg fluidisch verbindbar ist; wobei die Konzentratquelle einen oder mehrere gelöste Stoffe enthält und eine Vielzahl von Osmotikumquellen (404, 405) und eine oder mehrere Ionenkonzentratquellen (403) umfasst;
wobei die Vielzahl von Osmotikumquellen eine erste Osmotikumquelle (404), bestehend im Wesentlichen aus Glucose oder Dextrose, und eine zweite Osmotikumquelle (405), bestehend im Wesentlichen aus Icodextrin, umfassen;
ein Sterilisationsmodul (407), das mit dem Peritonealdialysaterzeugungsströmungsweg fluidisch verbindbar ist; und
einen integrierten Cycler (539), der mit dem Peritonealdialysaterzeugungsströmungsweg fluidisch verbunden ist,
und ferner umfassend eine Konzentratpumpe (515), die zwischen jeder der Vielzahl von Osmotikumquellen (404, 405) und dem Peritonealdialysaterzeugungsströmungsweg positioniert ist, für eine kontrollierte Zugabe von Fluid aus jeder der Vielzahl von Osmotikumquellen (404, 405) zu dem Peritonealdialysaterzeugungsströmungsweg.

2. System nach Anspruch 1, ferner umfassend ein oder mehrere Dialysatgefäße, die mit dem Peritonealdialysaterzeugungsströmungsweg stromabwärts des Sterilisationsmoduls (407) fluidisch verbindbar sind.

3. System nach einem der vorstehenden Ansprüche, wobei die Ionenkonzentratquelle ein oder mehrere Elemente aus der Gruppe, bestehend aus Natriumchlorid, Natriumlactat, Magnesiumchlorid, Calciumchlorid, Kaliumchlorid und Natriumbicarbonat, umfasst.

4. System nach einem der vorstehenden Ansprüche, ferner umfassend ein Steuersystem zum Steuern einer oder mehrerer Pumpen und Ventile, um eine Bewegung von Fluid durch das System zu steuern.

5. System nach Anspruch 6, wobei das Steuersystem einen Zeitgeber umfasst und wobei der Zeitgeber das Steuersystem veranlasst, zu einem zuvor bestimmten Zeitpunkt ein Peritonealdialysat zu erzeugen.

6. System nach Anspruch 4 oder 5, wobei das Steuersystem eine Benutzerschnittstelle (902) umfasst, wobei die Benutzerschnittstelle (902) das Steuersystem veranlasst, zu einem ausgewählten Zeitpunkt das Peritonealdialysat zu erzeugen.

7. System nach einem der vorstehenden Ansprüche, wobei das Sterilisationsmodul (407) eines oder mehrere aus der Gruppe umfasst, bestehend aus einem oder mehreren Ultrafiltern (518, 520), einer UV-Lichtquelle, einer Heizvorrichtung, einem Kurzzeithocherhitzungsmodul, einem mikrobiellen Filter und Kombinationen davon, wobei das Sterilisationsmodul (407) vorzugsweise die UV-Lichtquelle, die stromabwärts des Ultrafilters (518, 520) positioniert ist, umfasst.

8. System nach einem der vorstehenden Ansprüche, wobei das Wasserreinigungsmodul (402) eines oder mehrere umfasst, die aus der Gruppe ausgewählt sind, bestehend aus einer Sorptionskartusche (506), Aktivkohle, einem Umkehrosmosemodul, einem Kohlefilter und einem Nanofilter.

9. System nach einem der vorstehenden Ansprüche, wobei der integrierte Cycler (539) eine Heizvorrichtung und eine Pumpe umfasst.

10. System nach einem der vorstehenden Ansprüche, wobei der integrierte Cycler (539) ferner mindestens einen Sensor umfasst, der aus der Gruppe ausgewählt ist, bestehend aus einem Strömungsmesser, einem Drucksensor, einem Leitfähigkeitssensor und einem Temperatursensor.

11. System nach einem der vorstehenden Ansprüche, wobei der integrierte Cycler (539) eine Infusionsleitung (531) und eine Abflussleitung (533) umfasst.

12. System nach Anspruch 11, wobei die Abflussleitung (533) mit einem Abfallbehälter (537) fluidisch verbunden ist.

13. System nach einem der vorstehenden Ansprüche, wobei der integrierte Cycler (539) ferner einen Filter (522) in der Infusionsleitung (531) umfasst.

## Revendications

1. Système, comprenant :
une source d'eau (401) ;
une voie d'écoulement de génération de dialysat péritonéal ; dans lequel la voie d'écoulement de génération de dialysat péritonéal est reliée fluidiquement à la source d'eau (401) ;
un ou plusieurs modules de purification d'eau (402) reliés fluidiquement à la voie d'écoulement de génération de dialysat péritonéal ;
une source de concentré reliée fluidiquement à la voie d'écoulement de génération de dialysat péritonéal ; la source de concentré contenant un ou plusieurs solutés et comprenant de multiples sources d'agents osmotiques (404, 405) et une ou plusieurs sources de concentré d'ions (403) ;
dans lequel les multiples sources d'agents osmotiques comprennent une première source d'agents osmotiques (404) constituée essentiellement de glucose ou de dextrose et une seconde source d'agents osmotiques (405) constituée essentiellement d'icodextrine ;
un module de stérilisation (407) relié fluidiquement à la voie d'écoulement de génération de dialysat péritonéal ; et
un cycleur intégré (539) relié fluidiquement à la voie d'écoulement de génération de dialysat péritonéal,
et comprenant en outre une pompe de concentré (515) positionnée entre chacune des multiples sources d'agents osmotiques (404, 405) et la voie d'écoulement de génération de dialysat péritonéal pour l'ajout contrôlé de fluide à partir de chacune des multiples sources d'agents osmotiques (404, 405) à la voie d'écoulement de génération de dialysat péritonéal.

2. Système selon la revendication 1, comprenant en outre un ou plusieurs conteneurs de dialysat reliés fluidiquement à la voie d'écoulement de génération de dialysat péritonéal en aval du module de stérilisation (407).

3. Système selon quelconque revendication précédente, dans lequel la source de concentré d'ions comprend un ou plusieurs du groupe constitué de chlorure de sodium, lactate de sodium, chlorure de magnésium, chlorure de calcium, chlorure de potassium et bicarbonate de sodium.

4. Système selon l'une quelconque revendication précédente, comprenant en outre un système de commande pour commander une ou plusieurs pompes et vannes afin de contrôler le mouvement du fluide dans le système.

5. Système selon la revendication 6, dans lequel le système de commande comprend une minuterie, et dans lequel la minuterie amène le système de commande à générer un dialysat péritonéal à un moment prédéterminé.

6. Système selon la revendication 4 ou la revendication 5, dans lequel le système de commande comprend une interface utilisateur (902), dans lequel l'interface utilisateur (902) amène le système de commande à générer du dialysat péritonéal à un moment sélectionné.

7. Système selon l'une quelconque revendication précédente, dans lequel le module de stérilisation (407) comprend un ou plusieurs du groupe constitué d'un ou plusieurs ultrafiltres (518, 520), source de lumière UV, chauffage, module de pasteurisation flash, filtre microbien, et des combinaisons de ceux-ci, de préférence dans lequel le module de stérilisation (407) comprend la source de lumière UV positionnée en aval de l'ultrafiltre (518, 520).

8. Système selon l'une quelconque revendication précédente, dans lequel le module de purification d'eau (402) comprend un ou plusieurs éléments choisis parmi le groupe constitué d'une cartouche de sorbant (506), charbon actif, module d'osmose inverse, filtre à charbon et nanofiltre.

9. Système selon l'une quelconque revendication précédente, dans lequel le cycleur intégré (539) comprend un chauffage et une pompe.

10. Système selon l'une quelconque revendication précédente, dans lequel le cycleur intégré (539) comprend en outre au moins un capteur choisi parmi le groupe constitué d'un débitmètre, capteur de pression, capteur de conductivité et capteur de température.

11. Système selon l'une quelconque revendication précédente, dans lequel le cycleur intégré (539) comprend une ligne d'infusion (531) et une ligne de drainage (533).

12. Système selon la revendication 11, dans lequel la ligne de drainage (533) est reliée fluidiquement à un réservoir de déchets (537).

13. Système selon l'une quelconque revendication précédente, dans lequel le cycleur intégré (539) comprend en outre un filtre (522) dans la ligne d'infusion (531).
